# EUROPÄISCHE PATENTSCHRIFT

(11) **EP 1 117 652 B1**
(45) Veröffentlichungstag und Bekanntmachung des Hinweises auf die Patenterteilung: **26.05.2004**
(21) Anmeldenummer: 99968234.7
(22) Anmeldetag: 20.08.1999
(51) Int. Cl.: C07D 239/22, A61K 31/505, A61P 31/04

(54) **TAN-1057 DERIVATE**
TAN-1057 DERIVATIVES
DERIVES DE TAN 1057

(30) Priorität: 27.08.1998 DE 19838998
(43) Veröffentlichungstag der Anmeldung: 25.07.2001
(73) Patentinhaber: Bayer HealthCare AG, 51368 Leverkusen (DE)
(72) Erfinder: BRANDS, Michael, D-42329 Wuppertal (DE); ES-SAYED, Mazen, D-42115 Wuppertal (DE); HÄBICH, Dieter, D-42115 Wuppertal (DE); RADDATZ, Siegfried, D-51065 Köln (DE); KRÜGER, Joachim, D-42113 Wuppertal (DE); ENDERMANN, Rainer, D-42113 Wuppertal (DE); GAHLMANN, Reinhold, D-42327 Wuppertal (DE); KROLL, Hein-Peter, D-42115 Wuppertal (DE); GESCHKE, Frank-Ulrich, D-42115 Wuppertal (DE); DE MEIJERE, Armin, D-37077 Göttingen (DE); BELOV, Vladimir N., St.Petersburg, 196211 (RU); SOKOLOV, Victor, St.Petersburg, 195298 (RU); KOZHUSHKOV, Sergej, D-37075 Herberhausen (DE); KORDES, Markus, D-37075 Göttingen (DE)
(86) Internationale Anmeldenummer: PCT/EP1999/006124
(87) Internationale Veröffentlichungsnummer: WO 2000/012484

(56) Entgegenhaltungen:
- EP-A- 0 339 596
- WO-A-99/07685
- WILLIAMS R M ET AL: "Synthesis and antimicrobial evaluation of TAN-1057A/B analogs" J. ANTIBIOT. (JANTAJ,00218820);1998; VOL.51 (2); PP.189-201, XP002124285 Colorado State University;Department of Chemistry; Fort Collins; 80523; CO; USA (US) in der Anmeldung erwähnt
- C YUAN ET AL: "Total Synthesis of the Anti Methicillin-Resistant Staphylococcus aureus Peptide Antibiotics TAN-1057A-D" JOURNAL OF THE AMERICAN CHEMICAL SOCIETY,US,AMERICAN CHEMICAL SOCIETY, WASHINGTON, DC, Bd. 119, Nr. 49, Seite 11777-11784 XP002083366 ISSN: 0002-7863 in der Anmeldung erwähnt

## Beschreibung

Die vorliegende Erfindung betrifft neue Naturstoffderivate, Verfahren zu ihrer Herstellung, sie umfassende pharmazeutische Zusammensetzungen sowie ihre Verwendung bei der Behandlung von Erkrankungen bei Menschen oder Tieren.

Die EP-A-0339596 offenbart Antibiotika der Formel 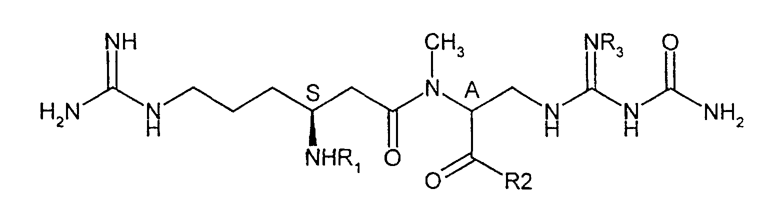 die durch Kultivieren eines Mikroorganismus der Gattung Flexibacter erhalten werden.

Spezifisch werden dort auch die folgenden Verbindungen beschrieben 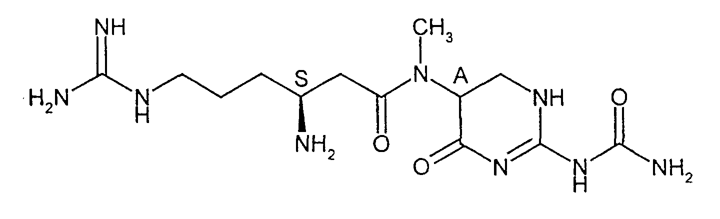 in denen das A-Kohlenstoffatom die S-Konfiguration aufweist (TAN-1057A) oder die R-Konfiguration aufweist (TAN-1057B). Y. Funabashi et al.; Tetrahedron 49, 13, 1993 beschreiben die chemische und strukturelle Charakterisierung von TAN-1057A und TAN-1057. B. N. Katayama et al.; J. Antibiotics, 46, 606, 1993 berichten über die Taxonomie der TAN-1057-erzeugenden Organismen sowie die biologischen Eigenschaften des TAN-1057. Totalsynthesen der TAN-1057-Verbindungen wurden von C. Yuan und R.M. Williams in J.

Am. Chem., Soc. 119, 11777, 1997 und A. de Meijere et al. in Eur. J. Org. Chem. 1998, 777 veröffentlicht. Erste Derivate der TAN-1057-Verbindungen wurden durch R.M. Williams in J. Antibiotics; 51, 189, 1998 beschrieben. Die Derivatisierungen betreffen jedoch weitgehend den cyclischen Amidinoharnstoff-Teil des Moleküls. So werden z.B. Derivate des Typs 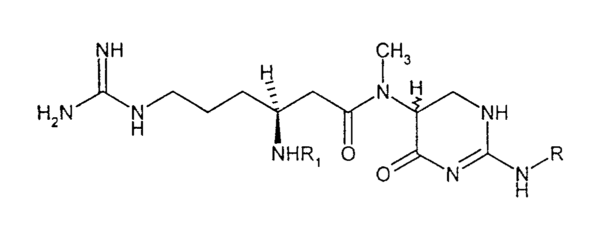 worin R Ac, COPh, COOMe, SO₂Me sowie CO₂CH₂Ph darstellt, beschrieben.

Die nach dem Prioritätstag dieser Anmeldung veröffentlichte WO 99/07685 offenbart am cyclischen Amidinoharnstoff-Teil des Moleküls acylierte und darüber hinaus phosphorylierte Derivate der allgemeinen Formel: 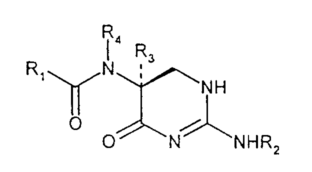 worin R₂ 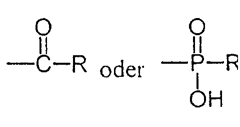 ist.

Lediglich zwei Derivatisierungen (J. Antibiotics; 51, 189, 1998) betreffen den (S)-β-Homoarginin-Teil:

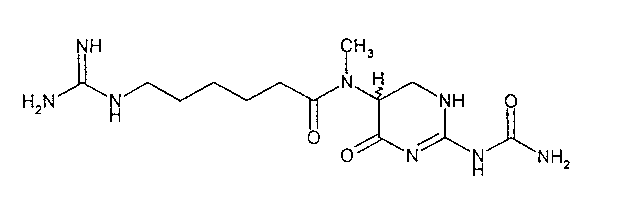

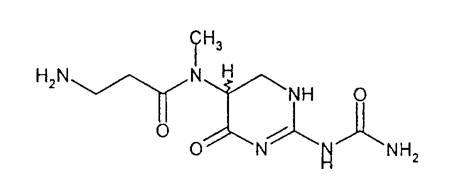

Diese Derivatisierungen führten jedoch zu einem vollständigen Verlust der biologischen Aktivität.

Die Erfinder der vorliegenden Erfindung stellten sich die Aufgabe, weitere Derivate der TAN-1057-Verbindungen zu synthetisieren, um ihre biologischen bzw. pharmakologischen Wirkungen zu untersuchen. Den Erfindern gelang es nach Überwindung schwieriger synthetischer Probleme weitere neue, im (S)-β-Homoarginin-Teil des TAN 1057 derivatisierte Verbindungen nach einem neuen allgemein anwendbaren Verfahren zu synthetisieren, die überraschender Weise bei vergleichbarer Wirksamkeit über eine wesentlich geringere Toxizität verfügen.

Gegenstand der vorliegenden Erfindung sind daher Verbindungen der allgemeinen Formel: 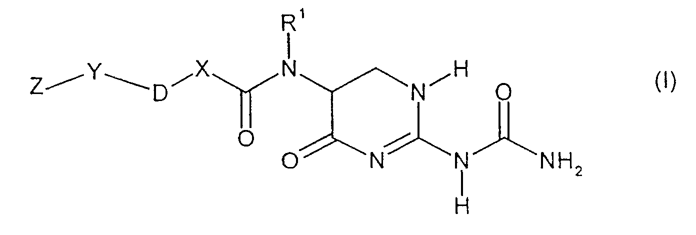 worin
- R¹: Wasserstoff oder (C₁-C₆)Alkyl ist,
- X: eine Gruppe der Formel -(CH₂)ₘ- darstellt, worin m 0, 1 oder 2 ist,
- D: ausgewählt wird aus Gruppen der Formeln D₁ bis D₃ 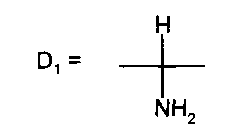 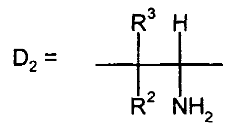 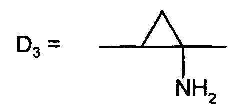 worin
R² Wasserstoff oder Hydroxy ist,
R³ Wasserstoff ist, oder
R² und R³ zusammen eine Oxogruppe bilden,
- Y: eine geradkettige oder verzweigtkettige (C₁-C₅)Alkandiyl-Gruppe, die gegebenenfalls durch Hydroxy oder Oxo substituiert sein kann, oder eine Gruppe der folgenden Formeln 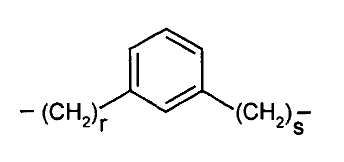 darstellt, worin r und s gleich oder verschieden sind und 0, 1 oder 2 sind,
- Z: eine Gruppe darstellt, die ausgewählt wird aus Gruppen der Formel 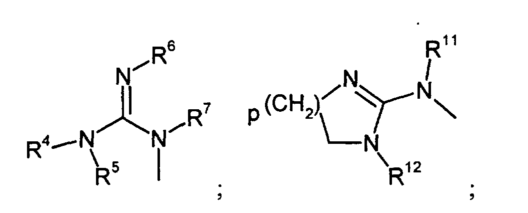 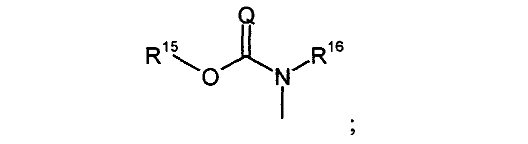 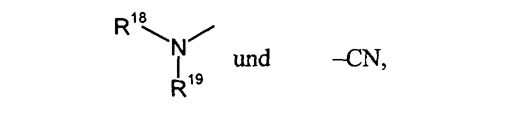 worin R⁴, R⁵, R⁶, R⁷, R¹¹, R¹², R¹⁵, R¹⁶, R¹⁸ und R¹⁹ jeweils unabhängig voneinander ausgewählt werden aus der Gruppe, die aus Wasserstoff, (C₁-C₆)Alkyl, (C₁-C₄)Alkanoyl, t-Butoxycarbonyl, Benzyloxycarbonyl und Benzyl besteht,
Q Sauerstoff oder Schwefel darstellt und
p 1, 2, oder 3 darstellt und mit Ausnahme der Verbindungen, worin
- R¹: Methyl ist, m 1 ist, D D₁ ist, Y -(CH₂)₃- ist, und Z eine Gruppe der Formel 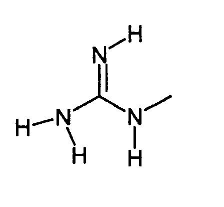 ist,
und pharmazeutisch verträgliche Salze davon.

Der TAN1057A/B entsprechende Fall, worin R¹ Methyl ist, m 1 ist, D D₁ ist, Y -(CH₂)₃- ist, und Z eine Gruppe der Formel 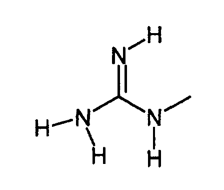 ist, der von den erfindungsgemäß beanspruchten Verbindungen ausgenommen ist, entspricht dem Fall, worin D D₂ ist, R² und R³ Wasserstoff sind, R¹ Methyl ist, m 1 ist, Y -(CH₂)₂- ist, und Z eine Gruppe der Formel 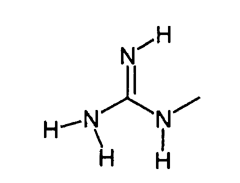 ist, der daher ebenfalls von den erfindungsgemäßen Verbindungen ausgenommen ist.

Gegenstand der vorliegenden Erfindung sind bevorzugt Verbindungen der allgemeinen Formel: 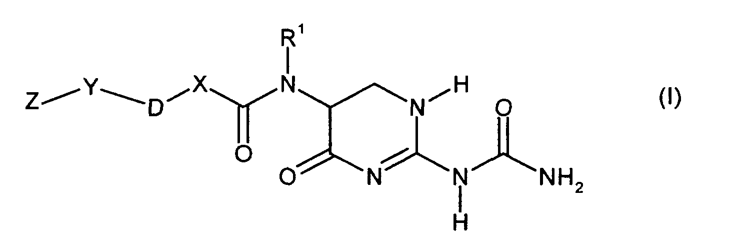 worin
- R¹: Wasserstoff oder (C₁-C₆)Alkyl ist,
- X: eine Gruppe der Formel -(CH₂)ₘ- darstellt, worin m 0, 1 oder 2 ist,
- D: ausgewählt wird aus Gruppen der Formeln D₁ bis D₃ 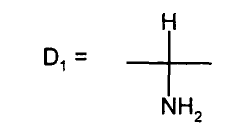 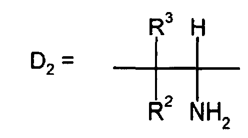 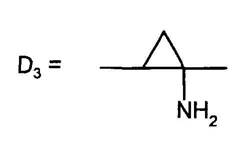 worin
R² Wasserstoff oder Hydroxy ist,
R³ Wasserstoff ist, oder
R² und R³ zusammen eine Oxogruppe bilden,
- Y: eine geradkettige oder verzweigtkettige (C₁-C₅)Alkandiyl-Gruppe, und die gegebenenfalls durch Hydroxy oder Oxo substituiert sein kann, oder eine Gruppe der folgenden Formeln 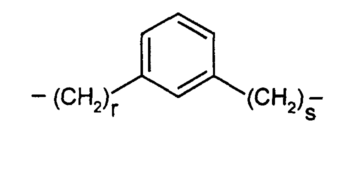 darstellt, worin r und s gleich oder verschieden sind und 0, 1 oder 2 sind,
- Z: eine Gruppe darstellt, die ausgewählt wird aus Gruppen der Formel 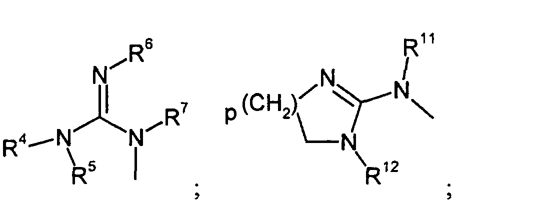 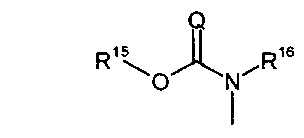 und 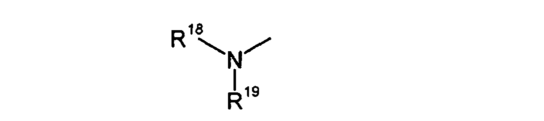 worin R⁴, R⁵, R⁶, R⁷, R¹¹, R¹², R¹⁵, R¹⁶, R¹⁸ und R¹⁹ jeweils unabhängig voneinander ausgewählt werden aus der Gruppe, die aus Wasserstoff, (C₁-C₆)Alkyl, (C₁-C₄)Alkanoyl, t-Butoxycarbonyl, Benzyloxycarbonyl und Benzyl besteht,
Q Sauerstoff oder Schwefel darstellt und
p 1, 2, oder 3 darstellt und
mit Ausnahme der Verbindungen, worin
- R¹: Methyl ist, m 1 ist, D D₁ ist, Y -(CH₂)₃- ist, und Z eine Gruppe der Formel 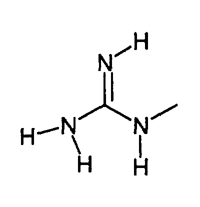 ist (entsprechend dem Fall, worin D D₂ ist, R² und R³ Wasserstoff sind, R¹ Methyl ist, m 1 ist, Y -(CH₂)₂- ist, und Z eine Gruppe der Formel 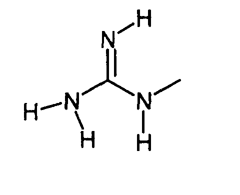
ist),
und pharmazeutisch verträgliche Salze davon.
m in der Gruppe der Formel -(CH₂)ₘ- für X ist bevorzugt 1 oder 2. Die Gruppe der Formel -(CH₂)ₘ- für X schließt daher bevorzugt eine Methylen- oder eine Ethylen (Ethan-1,2-diyl)-Gruppe ein. Besonders bevorzugt ist X eine Methylengruppe.

Eine geradkettige oder verzweigtkettige (C₁-C₅)Alkandiyl-Gruppe, die zusätzlich gegebenenfalls durch Hydroxy oder Oxo substituiert sein kann, in der Definition von Y schließt beispielsweise geradkettige (C₁-C₅)Alkandiyl-Gruppen ein, wie Methylen, Ethylen, Propan-1,3-diyl, Butan-1,4-diyl und Pentan-1,5-diyl. Bevorzugt sind geradkettige (C₁-C₄)Alkandiyl-Gruppen.

Dabei kann die Bindung der Gruppen an Z von beiden Seiten aus erfolgen.

Die Gruppen der Formel 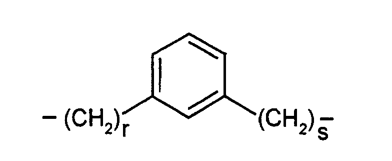 für Y schließen beispielweise symmetrische Reste, in denen r und s gleich sind, oder unsymmetrische Reste ein, wobei der symmetrische Rest, worin r und s 0 sind, also 1,3-Phenylen bzw. m-Phenylen bevorzugt ist.

In der allgemeinen Formel (I) wird Z ausgewählt aus Gruppen der Formel 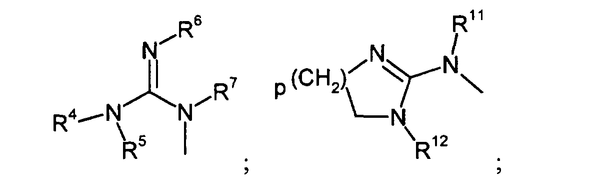 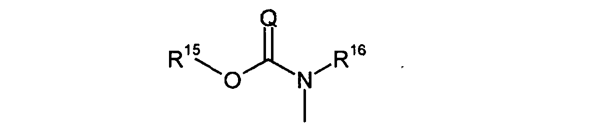 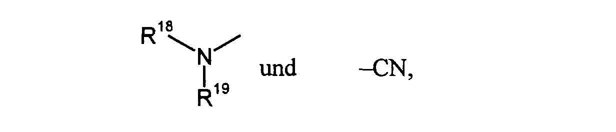 worin R⁴, R⁵, R⁶, R⁷, R¹¹, R¹², R¹⁵, R¹⁶, R¹⁸ und R¹⁹ jeweils unabhängig voneinander ausgewählt werden aus der Gruppe, die aus Wasserstoff, (C₁-C₆)Alkyl, (C₁-C₄)Alkanoyl, t-Butoxycarbonyl, Benzyloxycarbonyl und Benzyl besteht,
- Q: Sauerstoff oder Schwefel darstellt und
- p: 1,2, oder 3 darstellt und

Besonders bevorzugt ist Z eine Gruppe der Formel 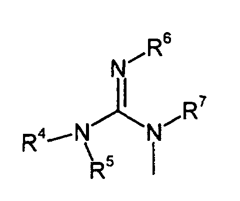 worin R⁴, R⁵, R⁶ und R⁷ wie oben definiert, bevorzugt Wasserstoff sind.

In einer weiteren bevorzugten Ausführungsform ist Z eine Gruppe der Formel 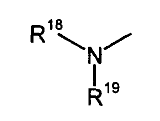 worin R¹⁸ und R¹⁹ wie oben definiert sind.

(C₁-C₆)Alkyl in den obigen Definitionen von R¹ und R⁴ bis R¹⁹ bedeutet eine geradkettige oder verzweigtkettige Alkylgruppe mit 1 bis 6 Kohlenstoffatomen wie z.B. Methyl, Ethyl, n-Propyl, iso-Propyl, n-Butyl, tert.-Butyl, sek.-Butyl, iso-Butyl, Pentyl und Hexyl, wobei (C₁-C₄)Alkyl-Gruppen bevorzugt sind und besonders Methyl bevorzugt ist.

Bevorzugt stehen R⁴ bis R¹⁹ für Wasserstoff.

(C₁-C₄)Alkanoyl in der Definition von R⁴ bis R¹⁹ steht beispielweise für Formyl, Acetyl, Propionyl und Butanoyl, wobei Formyl und Acetyl bevorzugt sind.

Q steht bevorzugt für Sauerstoff.

p steht bevorzugt für 1 oder 2.

In einer bevorzugten Ausführungsform der Erfindung steht die Gruppe D für

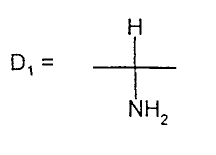

In einer weiteren bevorzugten Ausführungsform der Erfindung steht die Gruppe D für 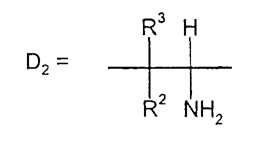 worin R² und R³ wie im oben definiert sind, bevorzugt Wasserstoff sind.

In einer bevorzugten Ausführungsform der Erfindung steht die Gruppe D für

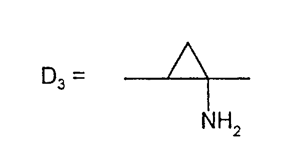

Darunter sind die Gruppen D₁ und D₂ bevorzugter und D₁ ist noch bevorzugter. Ferner ist der Fall, worin R² und R³ in D₂ beide Wasserstoff sind, bevorzugt.

Geeignete pharmazeutisch verträgliche Salze der Verbindungen der allgemeinen Formel (I) können konventionelle nicht-giftige Salze sein und schließen beispielsweise Salze mit Mineralsäuren, Carbonsäuren oder Sulfonsäuren ein. Solche Säureadditionssalze schließen organische Säuresalze (z.B. Acetate, Propionate, Laktate, Citrate, Benzoate, Trifluoracetate, Maleate, Tartrate, Fumarate, Methansulfonate, Ethansulfonate, Benzolsulfonate, Formiate, Toluolsulfonate, Naphthalindisulfonate, etc.) und anorganische Säuresalze (z.B. Hydrochloride, Hydrobromide, Hydroiodide, Sulfate, Nitrate, Phosphate, etc.) ein.

Die Verbindungen der allgemeinen Formel (I) können in Folge von Doppelbindungen und asymmetrischen Kohlenstoffatomen als Stereoisomere, wie cis-, trans-Isomere sowie Konfigurationsisomere wie Enantiomere oder Diastereomere vorliegen, und diese Isomere und Mischungen davon sind im Umfang der vorliegenden Erfindung enthalten.

Die Herstellung der erfindungsgemäßen Verbindungen erfolgt, in dem man Verbindungen der Formel (II) 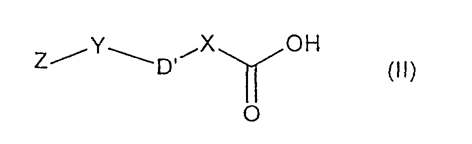 worin X, Y und Z wie oben definiert sind und D' ausgewählt wird aus Gruppen der Formeln D'₁ bis D'₃ 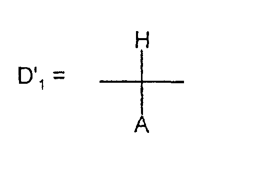 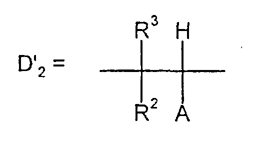 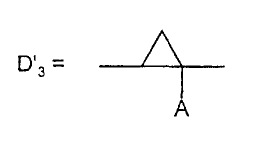 worin R² und R³ wie oben definiert sind und A eine konventionell geschützte Aminogruppe ist, mit Verbindungen der Formel (III) 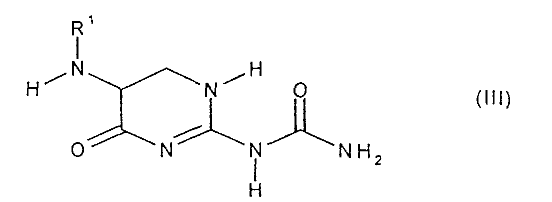 worin R¹ wie oben definiert ist, in Gegenwart von Kupplungsmitteln, wahlweise in Gegenwart von Basen umsetzt und in der geschützten Aminogruppe A die konventionelle Schutzgruppe nach an sich bekannten Verfahren abspaltet.

Die Verbindung der Formel (III) wird synthetisiert nach V.V. Sokolov, S.I. Kozhushkov, S. Nikolskaya, V.N. Belov, M. Es-Sayed, A. de Meijere, *Eur*. *J*. *Org. Chem*. **1998**, 777.

Als Verbindungen der Formel (II) für die Amidbildung werden entsprechend geschützte Aminosäuren, die z.B. durch Kettenverlängerung aus den α-Aminosäuren zugänglich sind (vgl. H.M.M. Bastiaans, A.E. Alewijnse, J.L. van der Baan, H.C.J. Ottenheijm, *Tetrahedron Lett*. **1994**, 35, 7659), eingesetzt.

Enthält die geradkettige oder verzweigtkettige (C₁-C₅)Alkandiyl-Gruppe für Y funktionelle Gruppen, wie Hydroxy-, Keto-, Ester- oder Amidfunktionen so erfolgt deren Einführung bzw. Aufbau nach Standardverfahren (s. z.B. Houben-Weyl, Methoden der organischen Chemie, Band XV/1 und 2.).

Beispielsweise erfolgt die Herstellung von 3-(Benzyloxycarbonylamino)-3-[3'-(Nbenzyloxycarbonylguanidino)phenyl]-propionsäure nach folgendem Schema 1. 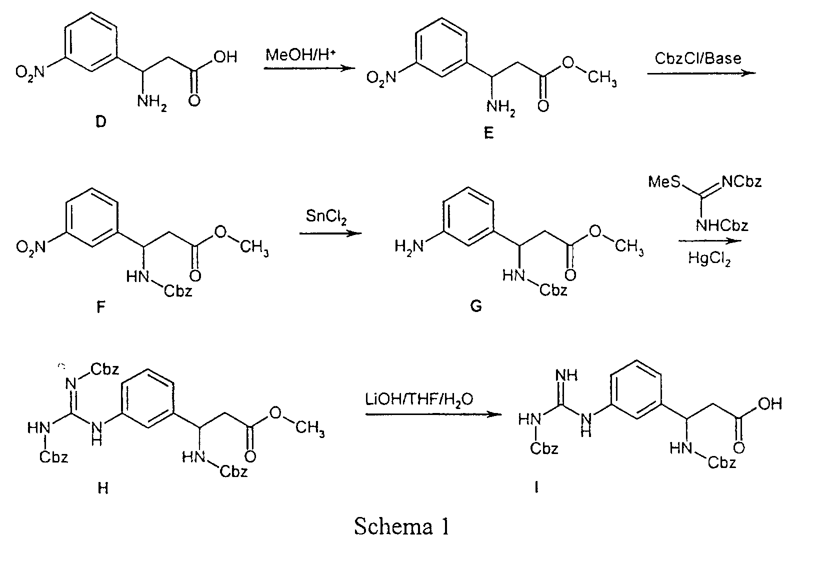
- Cbz:: 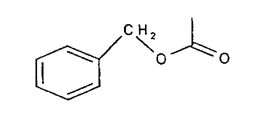
- MeOH:: Methanol
- THF:: Tetrahydrofuran
- H⁺:: Säure

Dabei wird die Verbindung D (E. Proft, F.-J. Becker, *J*. *prakt*. *Chemie* **1965**, *30*, 18) beispielsweise mit Methanol in Gegenwart von z.B. konzentrierter Schwefelsäure verestert. Die freie Aminogruppe wird im Zweiphasensystem Dichlormethan/Base, wie z.B. gesättigte wäßrige Natriumhydrogencarbonatlösung mittels Chlorkohlensäurebenzylester in das Benzylcarbamat überführt. Die Reduktion der aromatischen Nitrogruppe erfolgt mit Zinn(II)-chlorid. Anschließende Umsetzung mit Bis(benzyloxycarbonyl)-S-methylthioharnstoff (W. Su, Synth. Commun. 1996, 26, 407) in Gegenwart von Quecksilber(II)chlorid und Spaltung des Methylesters im Basischen ergibt die Carbonsäure I.

In den obigen Reaktionen können auch die unten aufgeführten allgemein anwendbaren Lösungsmittel, Säuren und Basen anstelle der hier genannten verwendet werden.

Die Darstellung der Verbindungen der Formel (II), in denen D D₃ ist, erfolgt beispielweise wie an folgendem Beispiel erläutert:

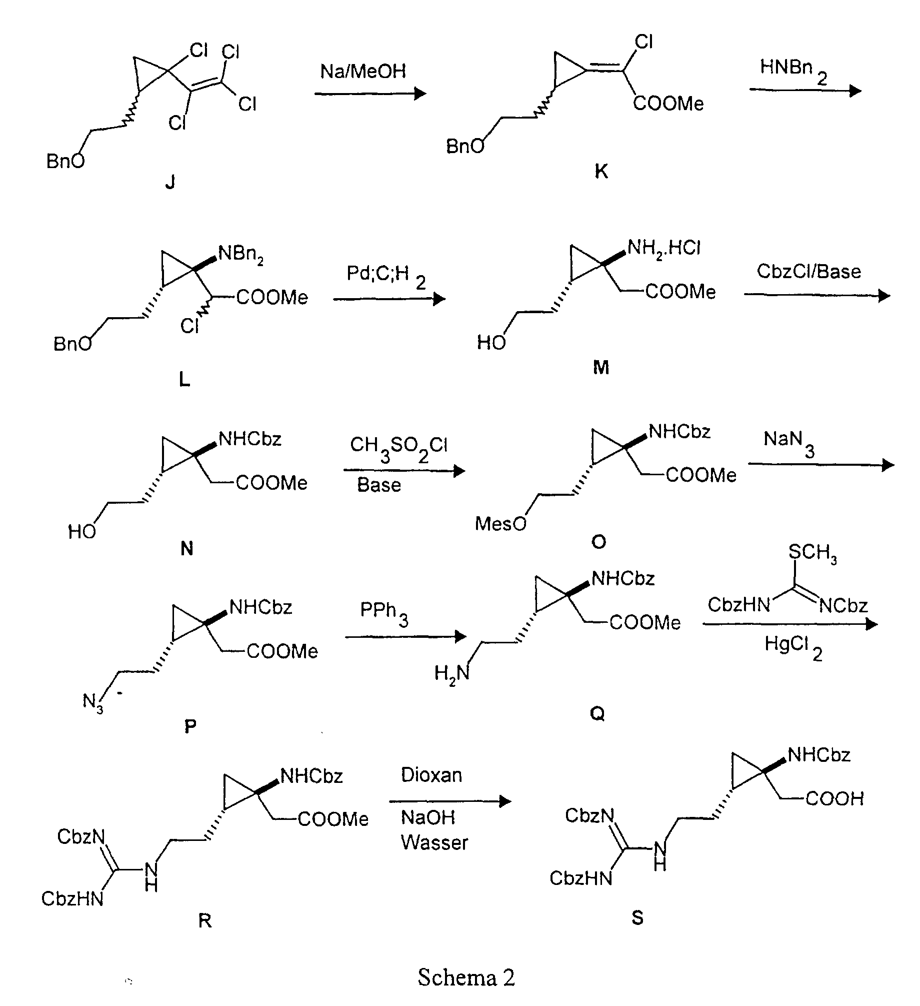

Dabei bedeuten:
Bn: -CH₂-Ph
Cbz: PhCH₂-O-C(O)-
Mes: CH₃SO₂-
Me: Methyl

Die Umsetzungen erfolgen dabei bevorzugt unter den im experimentellen Teil geschilderten Bedingungen.

Der Aufbau des Restes Z in der Säurekomponente der Formel (II) erfolgt prinzipiell durch Umsetzung eines S-Methylthioharnstoffderivats mit einer terminalen Aminogruppe. Diese Reaktion wird durch basische Reagenzien wie tertiäre Amine oder Natriumhydroxid gegebenenfalls in Gegenwart von Quecksilber(II)-chlorid vermittelt.

Ist Z eine Carbamatgruppe, so wird die Carbamatgruppe durch Umsetzung der terminalen Aminogruppe mit einem Chlorkohlensäureester in Gegenwart einer Base (z.B. ein tertiäres Amin oder Natriumhydroxid) erhalten (Schema 3). Die Säurefunktion kann dabei frei oder durch eine entsprechende Schutzgruppe (z.B. ein Alkylester) blockiert vorliegen.

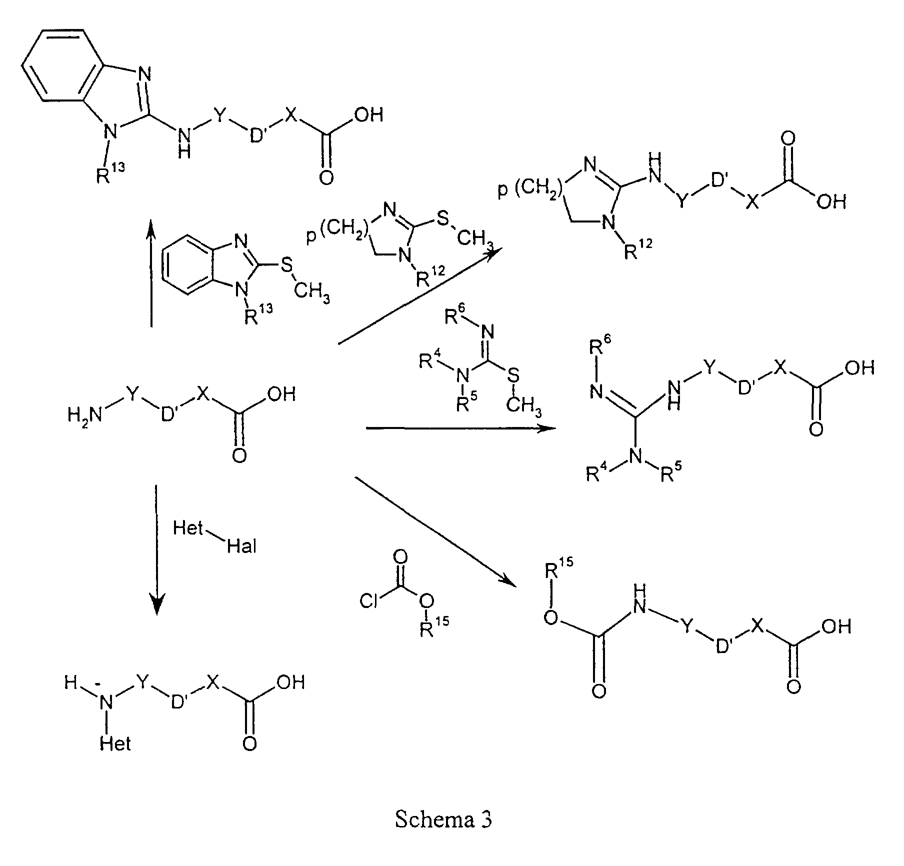

Die Substituenten bzw. Substituentengruppen sind dabei wie oben definiert, und Hal bedeutet Halogen, wie Fluor, Chlor, Brom und Iod.

Im Falle, daß R⁴, R⁵, R⁶, R⁷, R⁸, R⁹, R¹⁰, R¹¹, R¹², R¹³, R¹⁴, R¹⁵, R¹⁶, R¹⁷, R¹⁸ und R¹⁹ in den obigen Verfahren nicht Wasserstoff sondern (C₁-C₆)Alkyl, (C₁-C₄)Alkanoyl, t-Butoxycarbonyl, Benzyloxycarbonyl und Benzyl sind, erfolgt die Alkylierung der Aminogruppen nach üblichen Verfahren, beispielsweise mit Alkylierungsmitteln, wie Alkylhalogeniden, Sulfonsäureestem oder substituierten oder unsubstituierten Dialkyl- oder Diarylsulfonaten, wie Methyliodid oder Dimethylsulfat und die Einführung von (C₁-C₄)Alkanoyl, t-Butoxycarbonyl, Benzyloxycarbonyl und Benzyl, bei denen es sich um konventionelle Aminoschutzgruppen handelt, erfolgt nach üblichen Verfahren (vgl. T.W. Greene, P.G.M. Wuts, Protective Groups in Organic Synthesis, 2. Auflage, John Wiley and Sons, New York, 1991).

Die Abspaltung der konventionellen Aminoschutzgruppe in der konventionell geschützten Aminogruppe A erfolgt zweckmäßig nach den unten beschriebenen Verfahren.

Als Kupplungsmittel in der Reaktion der Verbindungen der Formel (II) und (III) können bekannte Reagenzien wie z.B. [O-(7-Azabenzotriazol-1-yl)-1,1,3,3-tetramethyl-uronium]hexafluorophosphat (HATU) oder [Bromo-tris-pyrrolidino-phosphonium]hexa-fluorophosphat (PyBroP) verwendet werden, da mit ihnen die Kupplung glatt und mit hohen Ausbeuten verläuft.

Als konventionelle Aminoschutzgruppe in der konventionell geschützten Aminogruppe A (also der konventionell geschützten -NH₂-Gruppe) können übliche in der Peptidchemie verwendete Aminoschutzgruppen eingesetzt werden. Hierzu gehören bevorzugt: Benzyloxycarbonyl, 2,4-Dimethoxybenzyloxycarbonyl, 4-Methoxybenzyloxycarbonyl, Methoxycarbonyl, Ethoxycarbonyl, tert.Butoxycarbonyl, Allyloxycarbonyl, Phthaloyl, 2,2,2-Trichlorethoxycarbonyl, Fluorenyl-9-methoxycarbonyl, Formyl, Acetyl, 2-Chloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl, 4-Methoxyphenyl oder Triphenylmethyl, wobei Benzyloxycarbonyl besonders bevorzugt ist.

Die Abspaltung der Aminoschutzgruppe in der konventionell geschützten Aminogruppe A erfolgt nach konventionellen Verfahren (vgl. T.W. Greene, P.G.M. Wuts, Protective Groups in Organic Synthesis, 2. Auflage, John Wiley and Sons, New York, 1991), und zwar vorzugsweise tert.-Butyloxycarbonyl mit Salzsäure in Dioxan, Fluorenyl-9-methoxycarbonyl mit Piperidin und Benzyloxycarbonyl durch Hydrogenolyse in Gegenwart von Katalysatoren wie z.B. mit Raney-Nickel, Palladium, Palladium auf Kohle oder Platin oder bevorzugt Palladium(II)chlorid. Gegebenenfalls in der Gruppe Z ebenfalls vorhandene Aminoschutzgruppen, wie z.B. Benzyloxycarbonyl können bei dieser Umsetzung ebenfalls abgespalten werden.

Die Reaktionen werden in der vorliegenden Erfindung in inerten organischen Lösungsmitteln durchgeführt, die sich unter den Reaktionsbedingungen nicht verändern. Hierzu gehören Ether wie Diethylether, 1,4-Dioxan oder Tetrahydrofuran, Halogenkohlenwasserstoffe wie Dichlormethan, Trichlormethan, Tetrachlormethan, 1,2-Dichlorethan, Trichlorethan oder Tetrachlorethan, Kohlenwasserstoffe wie Benzol, Toluol, Xylol, Hexan, Cyclohexan oder Erdölfraktionen, Alkohole wie Methanol, Ethanol oder iso-Propanol, Nitromethan, Dimethylformamid oder Acetonitril. Ebenso ist es möglich, Gemische der Lösungsmittel einzusetzen. Besonders bevorzugt sind Dichlormethan, Tetrahydrofuran, Dioxan, Methanol oder Dimethylformamid.

Die Reaktionen werden im allgemeinen in einem Temperaturbereich von 0°C bis 150°C, bevorzugt von 0°C bis 70°C durchgeführt. Die Umsetzungen können bei nomalem, erhöhtem oder bei erniedrigtem Druck durchgeführt werden (z.B. 0,5 bis 5 bar). Im allgemeinen arbeitet man bei Normaldruck.

Als Basen für die erfindungsgemäßen Verfahren können im allgemeinen Natriumoder Lithiumbistrimethylsilylamid, Alkalimetallhydroxide wie Natriumhydroxid, Lithiumhydroxid oder Kaliumhydroxid, Natriumhydrogencarbonat, Natriumhydrid oder organische (Trialkyl(C₁-C₆)amine) wie Triethylamin oder Heterocyclen wie 1,4-Diazabicyclo[5.4.0]undec-7-en (DBU), Pyridin, Diaminopyridin, Methylpiperidin, oder N-Methylmorpholin eingesetzt werden. Bevorzugt sind Lithiumhydroxid, Natriumhydrogencarbonat, Pyridin, Diisopropylethylamin und Triethylamin.

Eine geeignete Säure kann eine organische Säure einschließen (z.B. Ameisensäure, Essigsäure, Propionsäure, Trichloressigsäure, Trifluoressigsäure, etc.), eine anorganische Säure (z.B. Salzsäure, Bromwasserstoffsäure, Schwefelsäure, Chlorwasserstoff, Bromwasserstoff, Fluorwasserstoff, etc.) etc.

Die Naturstoffderivate der vorliegenden Erfindung weisen interessante pharmakologische Wirkungen auf. Insbesondere weisen die Verbindungen der vorliegenden Erfindung eine antibakterielle Wirkung auf und sind daher bei der Bekämpfung von bakteriellen Infektionen bei Menschen und Tieren wirksam. Desweiteren weisen die bekannten Verbindungen TAN-1057 A und B das Problem auf, daß die behandelten Keime rasch gegenüber diesen Verbindungen resistent werden, und man ist der Auffassung, daß die Verbindungen der vorliegenden Erfindung bei vergleichbarer antibakterieller Wirksamkeit weniger rasch zur Resistenzbildung führen können.

TAN1057 A,B zeigt ausgeprägte Toxizität in der Leber und Zellen des Immunsystems, die einen Einsatz dieser Substanz zur Therapie bakterieller Infektionen praktisch ausschließen. Alle Verbindungen der Erfindung zeigen gegenüber TAN 1057 A,B eine geringere Toxizität, was den therapeutischen Einsatz erlauben könnte.

### Bestimmung der Minimalen Hemmkonzentration (MHK)

Die MHK wurde im Flüssigdilutionstest bestimmt. Übernachtkulturen der Testkeime (S. aureus 133) in Isosensitest-Bouillon wurden 1:1000 in fötalem Kälberserum (FKS) oder Isosensitest-Bouillon verdünnt und mit Verdünnungen der Testsubstanzen (Verdünnungsstufen 1:2) inkubiert.

### Selektion TAN 1057 A,B-resistenter Bakterien

Bakterien des Stammes S. aureus 133 wurden in verschiedenen Konzentrationen von TAN 1057 A,B für 24 Stunden inkubiert. Bakterien, die bei höheren TAN 1057 A,B-Konzentrationen sichtbares Wachstum zeigten, wurden in neue Kulturflaschen mit verschiedenen TAN 1057 A,B-Konzentrationen überimpft und erneut inkubiert. Dieses Verfahren wurde über mehrere Tage wiederholt und schrittweise Bakterien mit erhöhter Resistenz gegen TAN 1057 A,B selektioniert. Die MHK hochresistenter S. aureus 133 Bakterien betrug > 100 µg/ml (Ausgangs-MHK: <0,1 µg/ml). Die Bakterien aus dieser Selektionsreihe mit einer MHK von <0,1, 0,8, 25, 100, und >100 µg/ml wurden für Testungen eingesetzt. Auf diesem Wege ist es möglich, TAN 1057 A,B-Derivate zu identifizieren, die gegen TAN 1057 A,B-resistente Zellen antibakterielle Wirkung zeigen.

### Toxikologische Untersuchungen

### Methodenbeschreibung:

Verträglichkeitsprüfungen der beispielhaften Verbindungen wurden an Kulturen von Eukaryontenzellen durchgeführt. Dabei wurden Hepatozyten (HepG2) und Mausmakrophagenzellen (J774.A1) als Indikator für organspezifische Toxizität eingesetzt. Die eingesetzte Maus-Makrophagen-Zellinie ist ein besonders sensitiver Indikator für toxische Effekte. Alle getesteten Derivate zeigten geringere Toxizität als TAN 1057 A,B.

### Testung mit HepG2-Zellen:

Die Vitalität und Funktionalität von humanen HepG2-Leberzellen wurden nach Behandlung mit beispielhaften Verbindungen geprüft. Jeweils 2 x 10⁴ - 1 x 10⁵ Zellen wurden in RPMI 1640 (Whittacker) mit 10% hitzeinaktiviertem fötalen Kälberserum (Gibco) für 40-48 Stunden bei 37°C inkubiert. In dem Inkubationsvolumen von 200 µl waren die Testsubstanzen in den Konzentrationen von 10, 2 und 0,4 µg/ml enthalten.

Die **Vitalität** wurde nach Zugabe von 20 µl Alamar Blue (Biosource, Art. Nr. DAL 1100) durch Messung der Fluoreszenz (Excitation: 544 nm, Emission: 590 nm) geprüft.

Die Funktionalität der HepG2-Zellen wurde durch Messung der Sekretion von apo B100 und α2-Macroglobulin nach Behandlung geprüft. Dazu wurden der Proteingehalt der Kulturüberstände nach Inkubation für 40-48 Stunden mittels ELISA ermittelt. Apo B100 wurde mittels Kaninchen ahLDL (1 mg/ml) gebunden und durch Peroxidase-konjugierte monoklonale Antikörper (ahLDL_POD) unter Zugabe von TMB/H₂O₂ als Substrat quantifiziert. Die Messung der optischen Dichte erfolgte bei 450 nm.

Für den Nachweis von α2-Macroglobulin wurde das ELISA-System von Biodesign (Art.Nr. H 45205M) eingesetzt. Die Quantifizierung erfolgte ebenfalls mit TMB/H₂O₂ als Substrat und einer Messung der optischen Dichte bei 450 nm.

### Testung mit J774.A1-Zellen:

Jeweils 1,2 x 10⁴ Zellen wurden in für 24 Stunden bei 37°C inkubiert. Testsubstanzen wurden in verschiedenen Konzentrationen zugegeben und die Zellen für weitere 72 Stunden inkubiert. Die Zellen wurden dann für 2 Stunden mit 50 µl Neutralrot-Lösung (Sigma, Nr. N 2889) behandelt, mit PBS-Medium gewaschen und einem Ethanol-Eisessig-Gemisch denaturiert.

Die Kulturen wurden im Elisa-Reader bei 540 nm und 630 nm gemessen. Die IC₅₀-Werte wurden extrapoliert und geben an, bei welcher Konzentration die Vitalität der Zellen im Vergleich zu unbehandelten Kontrollzellen gemessen an der Aufnahme von Neutralrot auf 50 % reduziert ist.

### Ergebnisse:

Die Kulturen wurden bei 37°C für 18-24 Stunden inkubiert. Die jeweils niedrigste Substanzkonzentration, bei der kein sichtbares Bakterienwachstum mehr auftrat, wurde als MHK definiert.

| Beispiel | MHK in FKS (S.aureus; µg/ml) |
|---|---|
| 1 | 0,2 |
| 2 | 50 |
| 3 | 100 |
| 4 | 0,4 |
| 5 | 0,1 |
| 6 | 0,8 |
| 7 | 0,8 |
| 8 | 1,6 |
| 9 | 1,6 |
| 10 | 3,2 |
| 11 | 3,2 |
| 12 | 6,3 |
| 13 | 12,5 |
| 14 | 12,5 |
| 15 | 100 |
| 16 | 100 |
| 17 | 6,3 |
| 18 | 12,5 |
| Vergleichsbeispiel | |
| TAN 1057 A, B (1:1-Gemisch der beiden Epimere) | 0,1 |

Die gegenüber TAN 1057 A,B intermediär- und hochresistenten S. aureus 133-Isolate wurden im Vergleich gegen die Derivate der Erfindung getestet. Bsp. 4 zeigte im MHK-Test mit dem intermediär TAN 1057 A,B-resistenten Isolat (MHK gegen TAN 1057 A,B = 0,8 µg/ml) eine bessere antibakterielle Wirkung (MHK gegen Beispiel 4 = 0,2 µg/ml).

### Testung der antibakteriellen Wirkung in vivo

Mäuse wurden mit 1x10⁶ Bakterien des Stammes *S*. *aureus 133* in 5 % Mucin (i.p.) infiziert und 30 Minuten nach der Infektion durch intravenöse Gabe der Testsubstanzen therapiert. Ohne Behandlung starben alle infizierten Tiere. Die therapeutischen Dosen für TAN 1057 A,B und die Verbindung des Beispiels 4, bei der alle infizierten Tiere überlebten (=ED₁₀₀), betrug für beide Substanzen 1 mg/Kg.

Die Verbindungen der Beispiele 1 und 2 zeigen bis zur höchsten Testkonzentration keine Hemmung in den Vitalitäts- und Funktionalitätstests (IC₅₀ > 10 µg/ml). Eine Hemmung (IC₅₀ 7 µg/ml) wurde für TAN 1057 A,B im α2-Macroglobulinassay gefunden.

Im Neutralrot-Vitalitätstest mit J774.A1-Zellen wurde ein IC₅₀-Werte für TAN 1057 A,B von 0.25 µg/ml bestimmt. Alle getesteten Derivate von TAN 1057 der vorliegenden Erfindung zeigen höhere IC₅₀-Werte, was die z.T. deutlich bessere Verträglichkeit der Verbindungen anzeigt.

### IC₅₀-Werte sind in der folgenden Tabelle aufgelistet:

| **Beispiel** | **IC**_{**50**}**-Wert** |
|---|---|
| TAN 1057 A,B (1:1-Gemisch der beiden Epimere) | 0,25 |
| 1 | 3 |
| 2 | 6 |
| 3 | 40 |
| 4 | 25 |
| 5 | 6 |
| 6 | 5,5 |
| 7 | 50 |
| 8 | 1,8 |

### Akute Toxizität von TAN 1057 A,B und der Verbindung von Beispiel 4

Die akute Toxizität der Substanzen wurde durch Bestimmung der Dosis ermittelt, bei der 50% der behandelten Mäuse überleben (=LD₅₀). Nach intraperitonealer Gabe der Testsubstanzen betrug die LD₅₀ für TAN 1057 A,B 100 mg/Kg. Die LD₅₀ für die Verbindung von Beispiel 4 war >400 mg/Kg.

Dieses Ergebnis spiegelt die deutlich geringere Toxizität der erfindungsgemäßen Verbindungen wieder. Die therapeutische Wirkung *in vivo* von TAN 1057 A,B und der Verbindung von Beispiel 4 war hingegen, wie oben beschrieben, vergleichbar.

Die erfindungsgemäßen Verbindungen der allgemeinen Formeln (I) weisen ein breites antibakterielles Spektrum, speziell gegen gram-positive Keime und einige gram-negative Bakterien sowie Corynebakterien auf. Diese Eigenschaften ermöglichen ihre Verwendung als chemotherapeutische Wirkstoffe in der Human- und Tiermedizin.

Mit ihrer Hilfe können gram-positive Keime (mit besonders guter Wirkung gegen Staphylokokken, einschließlich Methillicin-resistentem Staph. Aureus), gram-negative Bakterien (z.B. Moraxella catarrahlis) sowie Corynebakterien bekämpft sowie die durch diese Erreger hervorgerufenen Erkrankungen verhindert, gebessert und/oder geheilt werden.

Sie sind gut zur Prophylaxe und Chemotherapie von lokalen und systemischen Infektionen in der Human- und Tiermedizin geeignet, die durch solche Erreger hervorgerufen werden.

Zur vorliegenden Erfindung gehören pharmazeutische Zubereitungen, die neben nicht-toxischen, inerten, pharmazeutisch geeigneten Trägerstoffen oder Exzipienten eine oder mehrere erfindungsgemäße Verbindungen enthalten, oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung, vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg, Körpergewicht.

Die erfindungsgemäßen Verbindungen können zum Zweck der Erweiterung des Wirkungsspektrums und um eine Wirkungssteigerung zu erreichen, auch mit anderen Antibiotika kombiniert werden.

### Beispiele

Abkürzungen:

| | |
|---|---|
| DMF | *N*,*N*-Dimethylformamid |
| HATU | [O-(7-Azabenzotriazol-1-yl)-1,1,3,3-tetramethyluronium]hexafluorophosphat |
| PE | Petrolether (Spezialbenzin, Sdp. 40-80°C) |
| THF | Tetrahydrofuran |

### Beispiel 1

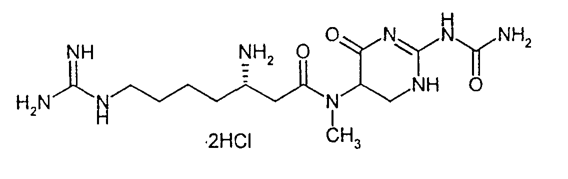

### (3'S,5R,S)-5-[N-Methyl-N-(3'-amino-7'-guanidinoheptanoyl)amino]-5,6-dihydro-2-ureido-4(1H)-pyrimidon-dihydrochlorid

Eine Lösung von 40 mg (0.216 mmol) (5R,S)-3,4,5,6-Tetrahydro-5-methylamino-2-ureidopyrimidin-4-on, 131 mg (0.261 mmol) (3S)-3-Benzyloxycarbonylamino-7-[bis-(N-benzyloxycarbonyl)guanidino]heptansäure, 80 mg (0.432 mmol) [O-(7-Azabenzotriazol-1-yl)-1,1,3,3-tetramethyluronium]hexafluorophosphat (HATU) und 53 mg (0.432 mmol) Diisopropylethylamin in 5 ml DMF wird bei 23°C 16 h gerührt. Anschließend wird das Lösungsmittel im Vakuum entfernt. Der Rückstand wird mit 2 M Salzsäure verrührt. Die wäßrige Phase wird vom Rückstand abdekantiert. Der Rückstand wird in Dichlormethan aufgenommen. Die organische Phase wird zweimal mit 2 M Salzsäure extrahiert, mit Natriumsulfat getrocknet und im Vakuum eingeengt. Man erhält 155 mg (93%) des Kupplungsprodukts als weißen Feststoff [MS (ESI): 772 (M+H)⁺]. Der Feststoff wird in 30 ml Methanol gelöst. Die Lösung wird mit 65 mg (0.369 mmol) Palladium(II)chlorid versetzt und 4 h unter einer Wasserstoffatmosphäre (Normaldruck) gerührt. Die Lösung wird filtriert und im Vakuum eingeengt. Der erhaltene Rückstand wird mit Diethylether verrührt und abfiltriert. Die Titelverbindung wird als beiger Feststoff erhalten (80 mg, 93%). ¹H-NMR (400 MHz, CD₃OD): 1.54 (m, 2H), 1.66 (m, 2H), 1.75 (m, 2H), 2.76 (dd, 1H), 2.98 (td, 1H), 3.17 + 3.36 (s, 3H), 3.21 (dd, 2H), 3.59 (m, 1H), 3.89 (m, 1H), 4.03 (m, 1H), 5.16 (m, 1H). MS (ESI) 370 (M+H)⁺.

### Beispiel 2

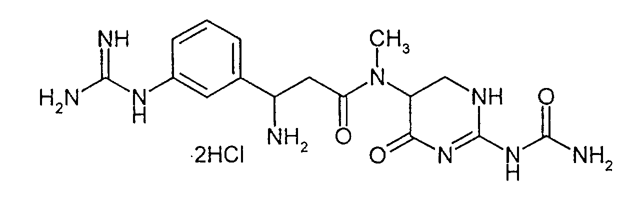

### (3'RS,5RS)-5-{N-Methyl-N-[3'-amino-3'-(3-guanidylphenyl)propionyl]amino}-5,6-dihydro-2-ureido-4(1H)-pyrimidon-dihydrochlorid

### (Verbindungen D bis I bezieht sich auf Schema 1)

Eine Lösung von 79.5 g ( 0.378 mol) D, 3-Amino-3-(3-nitrophenyl)propionsäure, in 2 l Methanol wird mit 200 ml Schwefelsäure (konz.) versetzt. Die Lösung wird 1 h auf Siedetemperatur erwärmt. Der größte Teil des Methanols wird im Vakuum entfernt, der Rückstand wird auf Eiswasser gegeben. Mittels Natriumcarbonat wird der pH Wert auf 8 eingestellt. Die wäßrige Phase wird mit Dichlormethan extrahiert. Trocknen der organischen Phase und Entfernen des Lösungsmittels im Vakuum ergibt den Ester E, 3-Amino-3-(3-nitrophenyl)propionsäuremethylester, (52.8 g, 62%) als weißen Feststoff. 10 g (44.6 mmol) E werden in 20 ml DMF gelöst. Man versetzt mit 12.3 g (88.2 mmol) Kaliumcarbonat und tropft 15.2 g (88.2 mmol) Chlorameisensäurebenzylester zu. Es wird 2 h bei 23°C gerührt. Man verdünnt mit 200 ml Toluol, wäscht dreimal mit Wasser, trocknet die organische Phase mit Natriumsulfat und engt im Vakuum zum farblosen Öl von 3-Benzyloxycarbonylamino-3-(3-nitrophenyl)propionsäuremethylester ein (12.6 g, 79 %). MS (DCI/NH₃): 376 (M+NH₄)⁺.

Eine Lösung aus 4 g (11.1 mmol) Verbindung F, 3-Benzyloxycarbonylamino-3-(3-nitrophenyl)propionsäuremethylester in 20 ml Ethanol wird bei 23°C zu einer Lösung von 12.6 g (55.8 mmol) Zinn(II)chloriddihydrat getropft. Man rührt 30 Minuten bei 80°C Badtemperatur und entfernt anschließend die Hauptmenge des Ethanols im Vakuum. Der Rückstand wird zwischen Wasser und Essigsäureethylester verteilt. Der pH-Wert der wäßrigen Phase wird durch Zugabe von Natriumhydrogencarbonat auf 8 eingestellt. Man filtriert durch eine 5 cm hohe Schicht von Celite und wäscht diese mit Essigsäureethylester. Nach Trennung der Phasen wird die wäßrige Phase dreimal mt Essigsäureethylester extrahiert. Die organische Phase wird getrocknet (Natriumsulfat). Entfernen des Lösungsmittels im Vakuum ergibt 3.8 g eines farblosen Öls G, 3-Benzyloxycarbonylamino-3-(3-aminophenyl)-propionsäure-methyl-ester, das noch Zinnsalze enthält. ¹H-NMR (200 MHz, CDCl₃): 2.87 (dd, 2H), 3.61 (s, 3H), 5.08 (m, 1H), 5.11 (s, 2H), 6.60 (m, 3 H), 7.11 (t, 1 H), 7.35 (m, 5 H). MS (DCI/NH₃): 346 (M+NH₄)⁺.

Eine Lösung aus 0.92 g (2.8 mmol) G, 3-Benzyloxycarbonylamino-3-(3-aminophenyl)-propionsäuremethylester und 1.0 g (2.8 mmol) Bis(benzyloxycarbonyl)-Smethylthioharnstoff in 10 ml DMF wird mit 1.56 ml (11.2 mmol) Triethylamin und 0.83 g (3.1 mmol) Quecksilber(II)chlorid versetzt. Man rührt 1 h bei 23°C, verdünnt mit 100 ml Essigsäureethylester und filtriert durch eine 5 cm hohe Celite Schicht. Man wäscht die organische Phase mit gesättigter wäßriger Natriumhydrogencarbonatlösung, trocknet (Natriumsulfat) und entfernt die flüchtigen Bestandteile im Vakuum. Der Rückstand wird an Kieselgel chromatographiert (Dichlormethan:Essigsäureethylester = 1:1). Man erhält 1.5 g (84 %) H, 3-Benzyloxycarbonyl-amino-3-[3-(N,N'-bisbenzyloxycarbonylguanidino)phenyl)-propionsäuremethylester als weißen Feststoff. ¹H-NMR (200 MHz, CDCl₃): 2.86 (m, 2H), 3.60 (s, 3H), 5.17 (m, 7H), 7.09 (d, 1H), 7.35 (m, 17H), 7.65 (d, 2H), 10.26 (s, br, 1H), 11.90 (s, br, 1H). MS (ESI): 639 (M+H)⁺.

Eine Lösung von 500 mg (0.78 mmol) von Verbindung H, Benzyloxycarbonylamino-3-[3-(N,N'-bisbenzyloxycarbonylguanidino)phenyl)propionsäuremethylester, in 7 ml THF und 3.5 ml Wasser wird mit 66 mg (1.56 mmol) Lithiumhydroxidmonohydrat versetzt. Man erwärmt über 16 h bei Siedetemperatur und entfernt anschließend das Lösungsmittel im Vakuum. Der Rückstand wird zwischen Wasser und Essigsäureethylester verteilt. Nach Trennung der Phasen wird die wäßrige Phase mit konzentrierter Salzsäure auf pH 1 eingestellt. Die wäßrige Phase wird zweimal mit Essigsäureethylester extrahiert, über Natriumsulfat getrocknet und einkondensiert. Der Rückstand wird an Kieselgel chromatographiert (Dichlormethan/Essigsäureethylester-Gradient). Man erhält 3-(Benzyloxycarbonylamino)-3-[3-(N-benyloxycarbonylguanidino)phenyl]-propionsäure (I) (270 mg, 70%) als farbloses Öl. ¹H-NMR (400 MHz, CD₃OD): 2.78 (m, 2H), 5.05 (s, 2H), 5.11 (m, 1H), 5.31 (s, 2H), 7.37 (m, 14H). MS (ESI): 491 (M+H)⁺.

Eine Lösung von 20 mg (0.108 mmol) (5R,S)-3,4,5,6-Tetrahydro-5-methylamino-2-ureidopyrimidin-4-on, 53 mg (0.108 mmol) der Säure I, 40 mg (0.216 mmol) [O-(7-Azabenzotriazol-1-yl)-1,1,3,3-tetramethyluronium]hexafluorophosphat (HATU) und 26 mg (0.216 mmol) Diisopropylethylamin in 5 ml DMF wird bei 23°C 16 h gerührt. Anschließend wird das Lösungsmittel im Vakuum entfernt. Der Rückstand wird mit 2 M Salzsäure verrührt. Die wäßrige Phase wird vom Rückstand abdekantiert. Der Rückstand wird in Dichlormethan aufgenommen. Die organische Phase wird zweimal mit 2 M Salzsäure extrahiert, mit Natriumsulfat getrocknet und im Vakuum eingeengt. Man erhält 70 mg (98%) des Kupplungsprodukts als weißen Feststoff [MS (ESI): 658 (M+H)⁺]. Dieser wird in 10 ml Methanol gelöst. Die Lösung wird mit 38 mg (0.213 mmol) Palladium(II)chlorid versetzt und 4 h unter einer Wasserstoffatmosphäre (Normaldruck) gerührt. Die Lösung wird filtriert und im Vakuum eingeengt. Der Rückstand wird mit Diethylether verrieben. Abdekantieren und Trocknen des Rückstands im Vakuum ergeben die Titelverbindung als farbloses Öl (28 mg, 57 %). ¹H-NMR (400 MHz, CD₃OD): 2.81-3.34 (m, 7H), 3.86 + 3.99 (m, 1H), 4.77 + 5.16 (m, 1H), 7.35-7.62 (m, 4H). MS (ESI) 390 (M+H)⁺.

### Beispiel 3

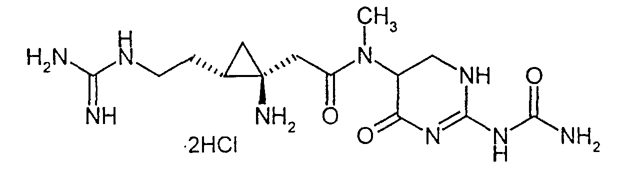

### 5-{N-Methyl-N-2'-[1-amino-2-(2-guanidinoethyl)cylopropyl]acetamino}-5,6-dihydro-2-ureido-4(1H)-pyrimidon-dihydrochlorid

Die Umsetzungen erfolgen gemäß obigem Schema 2, und die Bezeichnung der Verbindungen als J bis S erfolgt gemäß Schema 2.

Zu einer frisch bereiteten Lösung von 34.0 g Natrium (1.48 mol, 8 Äquiv.) in 250 ml Methanol wird bei 0°C langsam unter Rühren eine Lösung von 62.9 g (185 mmol) **J** 2-(2-Benzyloxyethyl)-1-chlor-1-(1,2,2-trichlorvinyl)cyclopropan in 50 ml wasserfreiem Methanol zugetropft. Anschließend wird 2 d unter Rückfluß erhitzt. Nach dem Abkühlen auf Raumtemp. wird mit 700 ml Wasser versetzt und mit Diethylether extrahiert. Die vereinigten organischen Phasen werden im Vakuum eingeengt und der Rückstand mit 300 ml Methanol aufgenommen. Man setzt 25 ml 10proz. Salzsäure zu und rührt das Reaktionsgemisch 45 min. Anschließend wird mit 300 ml gesättigter Natriumhydrogencarbonatlösung versetzt und mit Diethylether extrahiert. Die Etherextrakte werden über Calciumchlorid getrocknet. Nach dem Entfernen des Lösungsmittels im Vakuum erhält man ein dunkelbraunes Öl. Filtration durch 300 g Kieselgel (PE:Diethylether 10 : 1) ergibt 24.4 g (47%) K [2-(2-Benzyloxyethyl)-cyclo-propyliden]chloressigsäuremethylester in Form einer hellgelben Flüssigkeit als ein Gemisch zweier Diastereomeren im Verhältnis 1 : 1.6. - ¹³C-NMR (62.9 MHz, CDCl₃, zusätzl. DEPT), Isomer A: δ = 11.6 (-), 20.3 (+), 31.6 (-), 52.8 (+), 68.8 (-), 72.9 (-), 115.3 (C_{quart}), 127.49 (+), 127.53 (+), 128.3 (+), 138.3 (C_{quart}), 143.8 (C_{quart}), 162.4 (C_{quart}). - Isomer B: δ = 15.6 (-), 16.1 (+), 31.5 (-), 52.8 (+), 69.3 (-), 73.0 (-), 114.6 (C_{quart}), 127.49 (+), 127.53 (+), 128.3 (+), 143.3 (C_{quart}), 162.7 (C_{quart}). MS (70 eV), *m*/*z* (%): 280 (< 1) [M⁺], 245 (1) [M⁺ - Cl], 189 (2) [M⁺ - CH₂Ph], 91 (100) [CH₂Ph⁺]. - C₁₅H₁₇ClO₃ (280.8): ber. C 64.17, H 6.10; gef. C 63.24, H 5.97.

Eine Lösung von 11.98 g (42.7 mmol) **K** [2-(2-Benyloxyethyl)cyclopropyliden]-chlor-essigsäuremethylester in 100 ml wasserfreiem Methanol wird unter langsam mit 8.42 g (42.7 mmol) wasserfreiem *N*,*N*-Dibenzylamin versetzt. Nach 16 h Rühren bei Raumtemperatur wird die Lösung im Vakuum eingeengt. Säulenchromatographie an 300 g Kieselgel (PE:Diethylether 9 :1) ergibt 15.71 g (77 %) L, (E)-2-[2-(Benzyloxyethyl)-1-(N,N-dibenzyl-amino)cyclopropyl]-2-chloressigsäuremethylester, (hellgelbes Öl) als ein Gemisch zweier Diastereomeren im Verhältnis 1 : 1.6. - ¹³C-NMR (62.9 MHz, CDCl₃, zusätzl. DEPT), Isomer A: δ = 23.6 (-), 29.2 (+), 29.4 (-), 51.1 (C_{quart}), 52.8 (+), 56.6 (-), 62.1 (+), 69.8 (-), 72.8 (-), 126.5 (+), 126.6 (+), 127.6 (+), 128.3 (+), 128.7 (+), 128.8 (+), 138.4 (C_{quart}), 139.7 (C_{quart}), 169.1 (C_{quart}). - Isomer B: δ = 23.2 (-), 26.0 (+), 29.5 (-), 50.4 (C_{quart}), 52.9 (+), 56.6 (-), 64.3 (+), 69.8 (-), 72.9 (-), 126.5 (+), 126.6 (+), 127.6 (+), 128.3 (+), 128.7 (+), 128.8 (+), 138.4 (C_{quart}), 139.0 (C_{quart}), 169.5 (C_{quart}). MS (70 eV), *m*/*z* (%): 442 (<1) [M⁺ - Cl], 386 (<1) [M⁺ - CH₂Ph], 91 (100) [CH₂Ph⁺]. - C₂₉H₃₂ClNO₃ (478.0): ber. C 72.87, H 6.75; gef. C 72.53, H 6.84.

Ein Überdruckgefäß wird mit 100 ml Methanol und ca. 2.00 g Palladium auf Aktivkohle (10 %ig, 50 % Wasser) beschickt, mehrfach mit Wasserstoff gespült und 30 min bei 4.5 bar gerührt. Zu dem aktivierten Katalysator wird eine Lösung von 8.86 g (18.5 mmol) L, (E)-2-[2-(Benzyloxyethyl)-1-(N,N-dibenzylamino)cyclopropyl]-2-chloressigsäuremethylester, in 100 ml Methanol gegeben und bei 4.5 bar für 7 Tage bei Raumtemperatur gerührt. Dann wird der Katalysator mittels Filtration durch Celite abgetrennt und das Filtrat im Vakuum eingeengt. Der Rückstand wird in 110 ml gesättigter Natriumcarbonatlösung suspendiert und unter kräftigem Rühren im Eisbad mit 4.51 g (1.43 Äquiv.) Chlorameisensäurebenzylester versetzt und 5 h bei derselben Temperatur gerührt. Nach Extraktion mit Essigester und Trocknung über Magnesiumsulfat wird durch Säulenchromatographie an Kieselgel (Diethylether) gereinigt. Man erhält so 3.73 g (66%) N, (E)-2-[1-Amino-2-(hydroxyethyl)-cyclo-propyl]essigsäuremethylester-hydrochlorid. - ¹H-NMR (250 MHz, CDCl₃): δ = 0.39 (m_{c}, 1 H), 1.00-1.30 (m, 3H), 1.90-2.00 (m, 1H), 2.18 (d, *J*² = 17.3 Hz, 1H,), 3.02 (d, *J*² = 17.3 Hz, 1H), 3.66 (s, 3H), 3.74 (m_{c}, 2H), 5.05 (m_{c}, 2H), 5.92 (s, 1H), 7.31 (m_{c}, 5H). - ¹³C-NMR (62.9 MHz, CDCl₃, zusätzl. DEPT): δ = 18.0 (-), 24.4 (+), 32.1 (-), 33.6 (C_{quart}), 37.4 (-), 51.5 (+), 61.9 (-), 66.9 (-), 128.0 (+), 128.1 (+), 128.4 (+), 136.0 (C_{quart}), 157.1 (C_{quart}), 172.6 (C_{quart}). - MS (70 eV), *m*/*z* (%): 307 (<1) [M⁺], 276 (<1) [M⁺ - OCH₃], 172 (8) [M⁺ - OCOCH₂Ph], 91 (100) [CH₂Ph⁺].

Eine Lösung von 1.600 g (5.209 mmol) N, (E)-2-[1-Amino-2-(hydroxyethyl)cyclopropyl]-essigsäuremethylester-hydrochlorid, in 40 ml trockenem Dichlormethan wird auf 0°C abgekühlt, mit 1.02 g (2.0 Äquiv.) Triethylamin und 1.19 g (2.0 Äquiv.) Methansulfonsäurechlorid versetzt und 2 h bei gleicher Temperatur gerührt. Das Lösungsmittel wird im Vakuum entfernt, der Rückstand mit 50 ml Essigsäureethylester aufgenommen und mit 40 ml NaHCO₃-Lsg. gewaschen. Die wäßrige Phase wird mit Essigsäureethylester extrahiert. Nach Trocknung der organischen Phasen über Magnesiumsulfat und Entfernen des Lösungsmittels im Vakuum erhält man 2.010 g (quantitativ) von O, (E)-2-[1-Benzyloxycarbonylamino-2-(hydroxyethyl)cyclopropyl]-essigsäuremethylester, in Form eines leicht gelblichen Feststoffes. -¹H-NMR (250 MHz, CDCl₃): δ = 0.48 (m_{c}, 1H), 1.05-1.20 (m, 2H), 1.65-1.90 (m, 2H), 2.52 (d, 1H), 2.60 (d, 1H), 2.99 (s, 3H), 3.65 (s, 3H), 4.35 (m_{c}, 2H), 5.02 (s, 2H), 5.68 (s, 1H), 7.30 (m_{c}, 5H). - C₁₇H₂₃NO₇S (385.4): ber. C 52.98, H 6.01; gef. C 53.08, H 6.00.

2.010 g (5.209 mmol) **O** (E)-2-[1-Benzyloxycarbonylamino-2-hydroxyethyl)cyclopropyl]-essigsäuremethylester werden in 10 ml trockenem DMF gelöst, mit 1.69 g (5.0 Äquiv.) Natriumazid versetzt und 4 d bei Raumtemp. gerührt. Nach dem Entfernen des Lösungsmittels im Vakuum wird mit 100 ml Wasser versetzt und zweimal mit Essigsäureethylester extrahiert, über Natriumsulfat getrocknet und das Lösungsmittel im Vakuum entfernt. Säulenchromatographie an Kieselgel (PE:Diethylether 1 : 1 → DE) ergibt 1.540 g (89%) P, (E)-2-[2-(Azidoethyl)-1-(benzyloxycarbonylamino)cyclopropyl]essigsäuremethylester in Form eines farblosen Öles. - ¹H-NMR (250 MHz, CDCl₃): δ = 0.48 (m_{c}, 1H), 1.05-1.26 (m, 2H), 1.49 (m_{c}, 1H,), 1.75 (m_{c}, 1H), 2.54 (d, *J*² = 17 Hz, 1H), 2.69 (d, *J*² = 17 Hz, 1H), 3.42 (m_{c}, 2H), 3.68 (s, 3H), 5.05 (s, 2H), 5.60 (br. s, 1H), 7.33 (m_{c}, 5H). - ¹³C-NMR (62.9 MHz, CDCl₃, zusätzl. DEPT): δ = 19.3 (-), 23.2 (+), 29.2 (-), 33.5 (C), 36.6 (-), 50.8 (-), 51.8 (+), 66.5 (-), 128.0 (+), 128.5 (+), 136.2 (C_{quart}), 155.8 (C_{quart}), 172.3 (C_{quart}). - MS (DCI, NH₃), *m*/*z* (%): 350 (100) [M⁺ + NH₄], 333 (10) [M⁺ + H]. - C₁₆H₂₀N₄O₄ (332.4): ber. C 57.82, H 6.07; gef. C 57.54, H 5.87.

Eine Lösung von 1.51 g (4.54 mmol) P, (E)-2-[2-(Azidoethyl)-1-(benzyloxycarbonylamino)-cyclopropyl]essigsäuremethylester, in 5 ml THF wird bei Raumtemp. mit 1.19 g (1.0 Äquiv.) Triphenylphosphan und 82 µl (4.54 mmol) Wasser versetzt und 24 h gerührt. Das Lösungsmittel wird im Vakuum entfernt. Zum Rückstand werden 10 ml einer Mischung aus PE:Diethylether = 1 : 1 gegeben und so lange im Ultraschallbad behandelt, bis Triphenylphosphanoxid ausfälll. Letzteres wird abfiltriert und mehrfach mit insgesamt 100 ml des Lösungmittelgemisches gespült. Das Filtrat wird im Vakuum eingeengt und der Rückstand wird anschließend in 15 ml DMF gelöst und mit 1.63 g (1.0 Äquiv.) *N*,*N*'-Bis(benzyloxycarbonyl)-S-methylisothioharnstoff, 1.23 g (1.0 Äquiv.) Quecksilber(II)chlorid sowie 0.92 g (2.0 Äquiv.) Triethylamin versetzt. Nach 2 h Rühren wird über Celite filtriert und mit 150 ml Diethylether gespült. Nach dem Entfernen der Lösungsmittel im Vakuum wird der Rückstand mit 200 ml Dichlormethan aufgenommen und mit 100 ml Wasser gewaschen. Nach Trocknung über Magnesiumsulfat wird säulenchromatographisch an Kieselgel gereinigt (Diethylether). Man erhält so 1.82 g (65%) an **R**, (E)-2-{2-[N,N'-(Bisbenzyloxycarbonyl)guanidino]ethyl-1-(benzyloxycarbonylamino)cyclopropyl]essigsäure-methylester in Form eines glasigen Öles. - ¹H-NMR (250 MHz, CDCl₃): δ = 0.48 (m_{c}, 1H), 1.11 (m_{c}, 2H), 1.63 (m_{c}, 2H), 2.52 (d, *J*² = 17.3 Hz, 1H), 2.74 (d, *J*² = 17.3 Hz, 1H), 3.40-3.80 (m, 2H), 3.66 (s, 3H), 5.05-5.20 (m, 6H), 5.69 (s, 1H), 7.2-7.4 (m, 15H), 8.61 (br. s, 1H), 11.75 (br. s, 1H). - ¹³C-NMR (62.9 MHz, CDCl₃): δ = 19.2, 23.6, 28.8, 33.2, 36.7, 40.5, 51.6, 66.4, 67.0, 67.9, 127.7-128.6 (9 x C), 134.5, 136.2, 136.7, 153.5, 155.8, 163.6, 172.4.-C₃₃H₃₆N₄O₈ (616.7): ber. C 64.27, H 5.88; gef. C 64.57, H 6.07.

Eine Lösung von 300 mg (0.486 mmol) R, (E)-2-{2-[N,N'-(Bisbenzyloxycarbonyl)-guanidino]ethyl-1-(benzyloxycarbonylamino)cyclopropyl]essigsäuremethylester in 6 ml Dioxan wird bei Raumtemp. mit 5 ml 2 N Natronlauge versetzt. Nach 1 h wird mit 50 ml Wasser verdünnt und mit 50 ml Essigsäureethylester extrahiert. Durch Zugabe von 1 M Salzsäure wird mittels pH-Meter (Glaselektrode) auf pH = 5.4 angesäuert und mit Dichlormethan extrahiert. Beide organischen Extrakte werden getrennt mit je 30 ml gesättigter Ammoniumcarbonat-Lösung gewaschen, dann vereinigt und über Magnesiumsulfat getrocknet. Nach Entfernen des Lösungsmittels im Vakuum wird S (E)-2-{2-[N,N'-(Bisbenzyloxycarbonyl)-guanidino]ethyl-1-(benzyloxycarbonylamino)cyclopropyl]essigsäure als Öl erhalten. -¹H-NMR (250 MHz, CDCl₃): δ = 0.46 (m_{c}, 1H), 1.09 (m_{c}, 2H), 1.61 (m_{c}, 2H), 2.53 (d, *J*² = 17 Hz, 1H), 2.75 (d, *J*² = 17 Hz, 1H), 3.4-3.8 (m, 2H), 5.05-5.20 (m, 6H), 5.83 (s, 1H), 7.2-7.5 (m, 15H), 8.60 (s, 1H), 8.97 (s, 1H), 11-12 (br.S, 1H).-FAB-MS (Glycerin-Matrix), *m*/*z* (%): 625 (20) [M⁺ +Na], 603 (45) [M⁺ + H).

Die Kupplung von (5R,S)-3,4,5,6-Tetrahydro-5-methylamino-2-ureidopyrimidin-4-on und Säure S (E)-2-{2-[N,N'-(Bisbenzyloxycarbonyl)guanidino]ethyl-1-(benzyloxycarbonyl-amino)-cyclopropyl]essigsäure sowie die anschließende Abspaltung der Benzyloxycarbonyl-Schutzgruppen erfolgt wie bei Beispiel 1 beschrieben. Die Titelverbindung wird als amorpher Feststoff erhalten (Diastereomerenmischung). - ¹H-NMR (250 MHz, D₂O): 0.88 (m, 1H), 0.93 (m, 1H), 1.05 (m, 1H), 1.12 (m, 2H), 2.81-3.02 (m, 5H), 3.07 (m, 2H), 3.92 (m, 2H), 4.95 (m, 1H).

J 2-(2-Benzyloxyethyl)-1-chlor-1-(1,2,2-trichlorvinyl)cyclopropan wurde hergestellt nach: M. Es-Sayed, *Dissertation,* Universität Hamburg **1992**. - M. Kordes, *Diplomarbeit*, Universität Göttingen **1996**.

### Beispiel 4

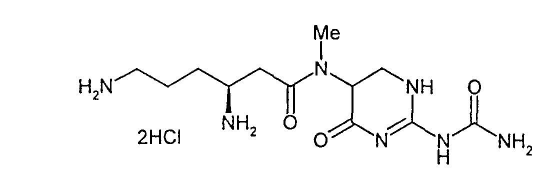

### (3'S,5R,S)-5-[N-Methyl-N-(3',6'-diaminohexanoyl)amino]-5,6-dihydro-2-ureido-4(1H)-pyrimidon-dihydrochlorid

Eine Lösung von 1.0 g (2.6 mmol) (3S)-3-Benzyloxycarbonylamino-6-tert-butyloxycarbonylaminohexansäure und 5 ml einer 4 M Chlorwasserstofflösung in Dioxan wird 30 min bei 23°C gerührt. Die flüchtigen Bestandteile werden im Vakuum entfernt. Man erhält einen öligen Rückstand, der ohne Reinigung weiter umgesetzt wird.

100 mg des Rückstands werden in 3 ml Dichlormethan suspendiert. Es werden 34 mg (0.316 mmol) Chlortrimethylsilan zugegeben. Nach 1 h Erwärmen auf 40°C wird die resultierend Lösung auf 0°Cgekühlt und nacheinander mit 45 mg (0.474 mmol) Chlorameisensäurebenzylester und 80 mg (0.789 mmol) Triethylamin versetzt. Es wird 1 h auf 40°C erwärmt. Anschließend werden 0.7 ml Methanol zugegeben. Nach Rühren bei 23°C für 10 min. werden die flüchtigen Bestandteile im Vakuum entfernt. Der Rückstand wird in Dichlormethan aufgenommen. Durch Waschen der organischen Phase mit 2 M Salzsäure, Trocknen der organischen Phase mit Natriumsulfat und Entfernen des Lösungsmittels wird der Rückstand mit Diethylether digeriert. Durch Abfiltrieren und Trocknen im Vakuum werden 100 mg (3S)-3,6-Bis-(benzyloxycarbonylamino)hexansäure als weißer Feststoff erhalten. ¹H-NMR (200 MHz, DMSO): 1.41 (m, 4H), 2.33 (d, 2H), 2.95 (m, 2H), 3.78 (m, 1H), 5.02 (s, 2 H), 7.22 (m, 2 H), 7.33 (m, 5 H). MS (DCI/NH₃): 432 (M+NH₄)⁺.

Die Kupplung von (5R,S)-3,4,5,6-Tetrahydro-5-methylamino-2-ureidopyrimidin-4-on und (3S)-3,6-Bis-(benzyloxycarbonylamino)hexansäure sowie die anschließende Abspaltung der Benzyloxycarbonyl-Schutzgruppen erfolgt wie bei Beispiel 1 beschrieben. Die Titelverbindung wird als amorpher Feststoff erhalten. ¹H-NMR (400 MHz, D₂O): 0.88 (m, 1H), 0.93 (m, 1H), 1.05 (m, 1H), 1.12 (m, 2H), 2.81-3.02 (m, 5H), 3.07 (m, 2H), 3.92 (m, 2H), 4.95 (m, 1H).

### Beispiel 5

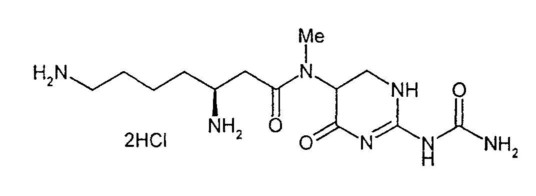

### (3'S,5R,S)-5-[N-Methyl-N-(3',7'-diaminoheptanoyl)amino]-5,6-dihydro-2-ureido-4(1H)-pyrimidon-dihydrochlorid

Die Kupplung von 86 mg (0.47 mmol) (5R,S)-3,4,5,6-Tetrahydro-5-methylamino-2-ureidopyrimidin-4-on und 200 mg (0.47 mmol) (3S)-3,7-Bis-(benzyloxycarbonylamino)heptan-säure sowie die anschließende Abspaltung der Benzyloxycarbonyl-Schutzgruppen erfolgt wie bei Beispiel 1 beschrieben. Die Titelverbindung wird als weißer Feststoff erhalten (57 mg, 30%). ¹H-NMR (400 MHz, CD₃OD): 1.55 (m, 2H), 1.74 (m, 4H), 2.75 (m, 1H), 2.96 (m, 3H), 3.14 (m, 3H), 3.58 (m, 1H), 3.89 (m, 1H), 4.01 (m, 1H), 5.14 (m, 1H).

### Beispiel 6

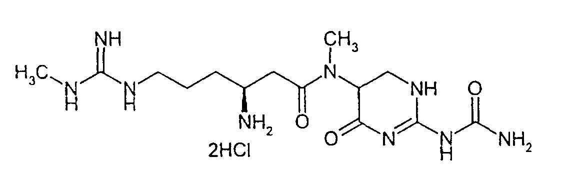

### (3'S,5R,S)-5-{N-Methyl-N-[3'-amino-6'-(N'-methylguanidino)hexanoyl]amino}-5,6-dihydro-2-ureido-4(1H)-pyrimidon-dihydrochlorid

Eine Lösung von 450 mg (1.1 mmol) (3S)-6-Amino-3-benzyloxycarbonylhexansäure-(2'-trimethylsilylethyl)ester und 400 mg (1.1 mmol) N,N'-Dibenzyloxycarbonyl-N-methyl-S-methylisothioharnstoff in 10 ml DMF wird bei Raumtemperatur mit 0.75 ml (5.37 mmol) Triethylamin und 320 mg (1.2 mmol) Quecksilber(II)-chlorid versetzt. Man rührt 17 h bei Raumtemperatur, filtriert vom ausgefallenen weißen Feststoff ab und entfernt die flüchtigen Bestandteile im Vakuum. Der Rückstand wird an Kieselgel chromatographiert (Dichlormethan:Essigsäureethylester 10:1 bis 3:1). Man erhält 470 mg (62%) (3S)-3-Benzyloxycarbonyl-6-[N,N'-bis-(benzyloxycarbonyl)-N-methylguanidino]hexansäure-(2'-trimethylsilylethyl)ester als farb-loses Öl. MS (ESI): 705 (M+H)⁺. Dieses Produkt wird in 10 ml THF gelöst und bei Raumtemperatur mit einer Lösung aus 421 mg (1.3 mmol) Tetrabutylammonimfluorid-trihydrat in 20 ml THF versetzt. Man rührt 2 h bei Raumtemperatur und gibt 50 ml Diethylether und 20 ml 2 M Salzsäure zu. Die Phasen werden getrennt und die wässrige Phase wird mit Diethylether extrahiert. Die vereinigten organischen Phasen werden über Na₂SO₄ getrocknet. Entfernen des Lösungsmittels im Vakuum gibt 250 mg (62 %) (3S)-3-Benzyloxycarbonyl-6-[N,N'-bis(benzyloxycarbonyl)-N-methylguanidino]hexansäure als farb-loses Öl. MS (ESI): 605 (M+H)⁺.

Die Kupplung von 76.5 mg (0.41 mmol) (5R,S)-3,4,5,6-Tetrahydro-5-methylamino-2-ureidopyrimidin-4-on mit der beschriebenen Säure sowie die anschließende Abspaltung der Benzyloxycarbonyl-Schutzgruppen erfolgt wie bei Beispiel 1 beschrieben. Die Titelverbindung wird als beiger Feststoff erhalten (180 mg, 99%). ¹H-NMR (400 MHz, CD₃OD): 1.70 (m, 4H), 2.72-2.94 (m, 2H), 2.84 (s, 3H), 3.14 (s, 3H), 3.23 (m, 2H), 3.61 (m, 1H), 3.90 (m, 1 H), 4.02 (dt, 1H), 5.15 (m, 1H).

### Beispiel 7

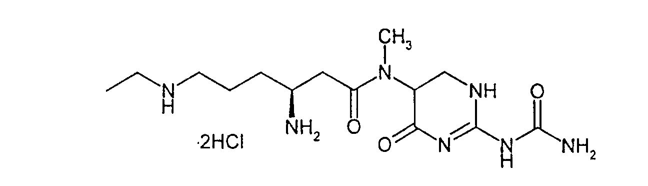

### (3'S,5R,S)-5-[N-Methyl-N-(3'-amino-6'-ethylaminohexanoyl)amino]-5,6-dihydro-2-ureido-4(1H)-pyrimidon-dihydrochlorid

1000 mg (3.0 mmol) (3S)-6-Amino-3-benzyloxycarbonylaminohexansäuremethylester werden in 5 ml 1,2-Dichlorethan vorgelegt und bei Raumtemperatur mit 250 µl (4.5 mmol) Acetaldehyd sowie 190 µl Essigsäure versetzt. Man rührt 30 min. bei Raumtempertur, kühlt auf 0°C und gibt 1601 mg (7.6 mmol) Natriumtriacetoxyborhydrid zu. Man rührt 20 h bei Raumtemperatur, verdünnt mit 30 ml Dichlormethan und extrahiert mit 1 M Salzsäure. Die wässrige Phase wird mit Natriumhydrogencarbonatlösung auf pH 9 gebracht und dreimal mit je 30 ml Essigsäureethylester extrahiert. Die vereinigten organischen Phasen werden mit Na₂SO₄ getrocknet und das Lösungsmittel wird im Vakuum abdestilliert. Man erhält 640 mg (66 %) (3S)-3-Benzyloxycarbonylamino-6-ethylaminohexansäuremethylester als farbloses Öl. ¹H-NMR (200 MHz, DMSO): 0.97 (t, 3H), 1.37 (m, 4H), 2.42 (m, 6H), 3.56 (s, 3H), 3.78 (m, 1H), 5.02 (s, 2H), 7.35 (m, 6H). MS (DCI/NH₃): 323 (M+H)⁺.

Das beschriebene Produkt (630 mg, 1.95 mmol) wird in 10 ml Dichlormethan gelöst und bei 0°C mit 300 µl (2.15 mmol) Triethylamin und 310 µl (2.15 mmol) Chlorameisensäurebenzylester versetzt. Man rührt 16 h bei Raumtemperatur, wäscht die organische Phase zweimal mit Wasser, trocknet über Na₂SO₄ und entfernt das Lösungsmittel im Vakuum. Der Rückstand wird an Kieselgel chromatographiert (Essigsäureethylester/Cyclohexan 1:1). Man erhält 515 mg (58%) (3S)-3-Benzyloxycarbonylamino-6-[(benzyloxycarbonyl)ethylamino]hexansäuremethylester als weißen Feststoff. ¹H-NMR (200 MHz, DMSO): 1.02 (t, 3H), 1.40 (m, 4H), 2.42 (m, 2H), 3.18 (m, 4H), 3.56 (s, 3H), 3.82 (m, 1H), 5.01 (s, 2H), 5.06 (s, 2H), 7.25 (m, 1H), 7.35 (m, 10H). MS (ESI): 457 (M+H)⁺.

Das beschriebene Produkt (510 mg, 1.12 mmol) wird in 4 ml Dichlormethan gelöst und bei Raumtemperatur mit 158 mg (1.30 mmol) Kaliumtrimethylsilanolat versetzt. Man rührt 16 h bei Raumtemperatur, verdünnt mit 20 ml Dichlormethan, wäscht mit 1 M Salzsäure, trocknet die organische Phase über Na₂SO₄ und entfernt die flüchtigen Komponenten im Vakuum. Es resultieren 463 mg (94 %) (3S)-3-Benzyloxycarbonylamino-6-[(benzyloxycarbonyl)-ethyl]aminohexansäure als weißer Feststoff. ¹H-NMR (200 MHz, DMSO): 1.02 (t, 3H), 1.40 (m, 4H), 2.35 (m, 2H), 3.20 (m, 4H), 3.81 (m, 1H), 5.00 (s, 2H), 5.05 (s, 2H), 7.25 (m, 1H), 7.33 (m, 10H). MS (ESI): 443 (M+H)⁺.

Die Kupplung von 42 mg (0.27 mmol) (5R,S)-3,4,5,6-Tetrahydro-5-methylamino-2-ureidopyrimidin-4-on und 100 mg (0.27 mmol) der beschriebenen Säure sowie die anschließende Abspaltung der Benzyloxycarbonyl-Schutzgruppen erfolgt wie bei Beispiel 1 beschrieben. Die Titelverbindung wird als weißer Feststoff erhalten (60 mg, 64 %). ¹H-NMR (400 MHz, CD₃OD): 1.32 (t, 3H), 1.83 (m, 4H), 2.80 (dd, 1H), 2.95-3.18 (m, 5H), 3.19 (s, 3H), 3.61 (m, 1H), 3.90 (ddd, 1H), 4.03 (dt, 1H), 5.18 (m, 1H). MS (ESI): 342 (M+H)⁺.

### Beispiel 8

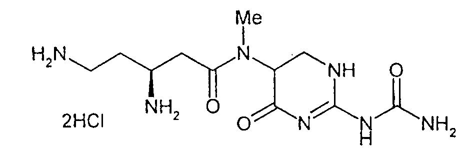

### (3'S,5R,S)-5-[N-Methyl-N-(3',5'-diaminopentanoyl)amino]-5,6-dihydro-2-ureido-4(1H)-pyrimidon-dihydrochlorid

Die Kupplung von 46 mg (0.25 mmol) (5R,S)-3,4,5,6-Tetrahydro-5-methylamino-2-ureidopyrimidin-4-on und 100 mg (0.25 mmol) (3S)-3,5-Bis-(benzyloxy-carbonylamino)pentansäure sowie die anschließende Abspaltung der Benzyloxycarbonyl-Schutzgruppen erfolgt wie bei Beispiel 1 beschrieben. Die Titelverbindung wird als weißer Feststoff erhalten (25 mg, 27%). ¹H-NMR (400 MHz, CD₃OD): 2.10 (m, 2H), 2.86 (dd, 1H), 3.04 (m, 1H), 3.10 (dd, 2H), 3.18 (s, 3H), 3.72 (m, 1H), 3.89 (ddd, 1H), 4.02 (dt, 1H), 5.19 (m, 1H).

### Beispiel 9

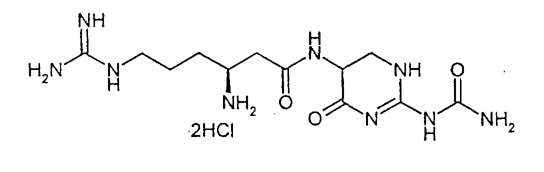

### (3'R,5R,S)-5-[N-(3'-amino-6'-guanidinohexanoyl)amino]-5,6-dihydro-2-ureido-4(1H)-pyrimidon-dihydrochlorid

Die Darstellung der Titelverbindung und der benötigten Komponente (5R,S)-3,4,5,6-Tetrahydro-5-amino-2-ureidopyrimidin-4-on erfolgten analog zu beschriebenen Synthesen (vgl. V.V. Sokolov, S.I. Kozhushkov, S. Nikolskaya, V.N. Belov, M. Es-Sayed, A. de Meijere, *Eur*. *J*. *Org*. *Chem*. **1998**, 777). ¹H-NMR (200 MHz, D₂O): 1.45-1.65 (m, 4H), 2.55-2.70 (m, 2H), 3-05-3.13 (m, 2H), 3.55 (m, 1H), 3.62 (dd, 1H), 3.71 (dd, 1H), 4.87 (dd, 1H).

### Beispiel 10

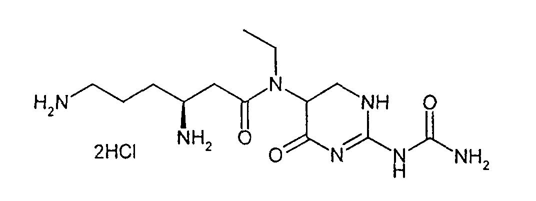

### (3'S,5R,S)-5-[N-Ethyl-N-(3',6'-diaminohexanoyl)amino]-5,6-dihydro-2-ureido-4(1H)-pyrimidon-dihydrochlorid

Die Kupplung von 120 mg (0.60 mmol) (5R,S)-3,4,5,6-Tetrahydro-5-ethylamino-2-ureidopyrimidin-4-on (synthetisiert in Analogie zur Methylverbindung: vgl. V.V. Sokolov, S.I. Kozhushkov, S. Nikolskaya, V.N. Belov, M. Es-Sayed, A. de Meijere, *Eur*. *J*. *Org*. *Chem*. **1998**, 777) und 250 mg (0.60 mmol) (3S)-3,6-Bis-(benzyloxycarbonylamino)hexansäure sowie die anschließende Abspaltung der Benzyloxycarbonyl-Schutzgruppen erfolgt wie bei Beispiel 1 beschrieben. Die Titelverbindung wird als amorpher Feststoff erhalten (115 mg, 48%). ¹ H-NMR (400 MHz, CD₃OD): 1.26 (t, 3H), 1.78 (m, 4H), 2.62-2.90 (m, 2H), 2.98 (m, 2H), 3.49 (m, 1H), 3.63 (m, 2H), 3.89 (m, 1H), 4.08 (m, 1H), 4.62 (m, 1H). MS (ESI): 328 (M+H)⁺.

### Beispiel 11

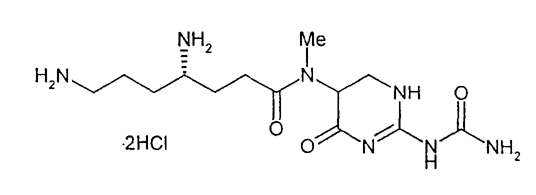

### (4'S,5R,S)-5-[N-Methyl-N-(4',7'-diaminoheptanoyl)amino]-5,6-dihydro-2-ureido-4(1H)-pyrimidon-dihydrochlorid

Die Synthese von (4S)-4,7-Bis-(benzyloxycarbonylamino)heptansäure erfolgt aus (3S)-3,6-Bis-(benzyloxycarbonylamino)hexansäure (vgl. Beispiel 4) in Analogie zu einem Literaturbeispiel (H.M.M. Bastiaans, A.E. Alewijnse, J.L. van der Baan, H.C.J. Ottenheijm, *Tetrahedron Lett*. **1994**, 35, 7659). Die Kupplung von 22 mg (0.12 mmol) (5R,S)-3,4,5,6-Tetrahydro-5-methylamino-2-ureidopyrimidin-4-on und 50 mg (0.12 mmol) der entsprechenden Säure sowie die anschließende Abspaltung der Benzyloxycarbonyl-Schutzgruppen erfolgt wie bei Beispiel 1 beschrieben. Die Titelverbindung wird als weißer Feststoff erhalten (25 mg, 52 %). MS (ESI): 328 (M+H)⁺.

### Beispiel 12

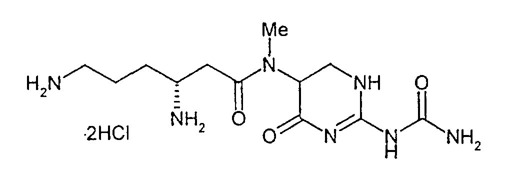

### (3'R,5R,S)-5-[N-Methyl-N-(3',6'-diaminohexanoyl)amino]-5,6-dihydro-2-ureido-4(1H)-pyrimidon-dihydrochlorid

Die Kupplung von 112 mg (0.60 mmol) (5R,S)-3,4,5,6-Tetrahydro-5-methylamino-2-ureidopyrimidin-4-on und 250 mg (0.60 mmol) (3R)-3,6-Bis-(benzyloxycarbonylamino)hexansäure sowie die anschließende Abspaltung der Benzyloxycarbonyl-Schutzgruppen erfolgt wie bei Beispiel 1 beschrieben. Die Titelverbindung wird als beiger Feststoff erhalten (204 mg, 88%). ¹ H-NMR (400 MHz, CD₃OD): 1.78 (m, 4H), 2.80 (m, 2H), 2.96 (m, 2H), 3.17 (s, 3H), 3.62 (m, 1 H), 3.92 (m, 1H), 4.03 (m, 1H), 5.18 (m, 1H).

### Beispiel 13

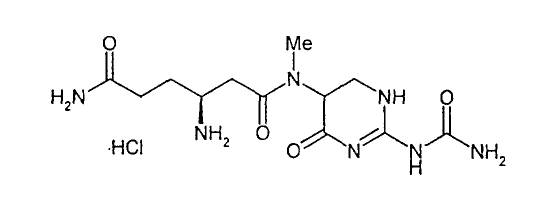

### (3'R,5R,S)-5-[N-Methyl-N-(3'-amino-5'-carbamoyl-pentanoyl)amino]-5,6-dihydro-2-ureido-4(1H)-pyrimidon-dihydrochlorid

Die Kupplung von 157 mg (0.85 mmol) (5R,S)-3,4,5,6-Tetrahydro-5-methylamino-2-ureidopyrimidin-4-on und 250 mg (0.85 mmol) (3R)-3-(Benzyloxycarbonylamino)-5-carbamoylpentansäure sowie die anschließende Abspaltung der Benzyloxycarbonyl-Schutzgruppen erfolgt wie bei Beispiel 1 beschrieben. Die Titelverbindung wird als beiger Feststoff erhalten (81 mg, 26%). ¹ H-NMR (400 MHz, CD₃OD): 1.94 (m, 2H), 2.45 (m, 2H), 2.72 (m, 1H), 2.91 (m, 1H), 3.09 (s, 3H), 3.61 (m, 1H), 3.78 (ddd, 1H), 3.94 (m, 1H), 5.15 (m, 1H).

### Beispiel 14

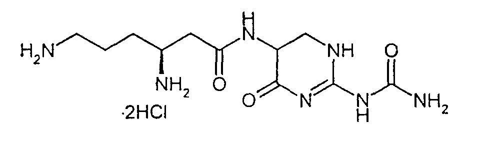

### (3'R,5R,S)-5-[N-(3',6'-Diaminohexanoyl)amino]-5,6-dihydro-2-ureido-4(1H)-pyrimidon-dihydrochlorid

Die Kupplung von 62 mg (0.36 mmol) (5R,S)-3,4,5,6-Tetrahydro-5-amino-2-ureidopyrimidin-4-on (vgl. Beispiel 10) und 150 mg (0.36 mmol) (3S)-3,6-Bis-(benzyloxycarbonylamino)hexansäure sowie die anschließende Abspaltung der Benzyloxycarbonyl-Schutzgruppen erfolgt wie bei Beispiel 1 beschrieben. Die Titelverbindung wird als weißer Feststoff erhalten (110 mg, 82 %). ¹H-NMR (400 MHz, CD₃OD): 1.82 (m, 4H), 2.75 (dd, 1H), 2.80 (dd, 1H), 2.99 (m, 2H), 3.62 (m, 1H), 3.78 (m, 1H), 3.94 (m, 1H), 5.02 (m, 1H).

### Beispiel 15

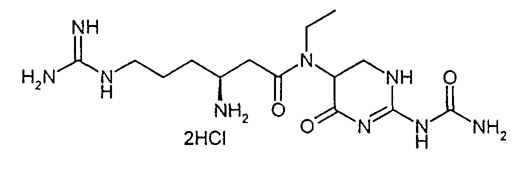

### (3'S,5R,S)-5-[N-Ethyl-N-(3'-amino-6'-guanidinohexanoyl)amino]-5,6-dihydro-2-ureido-4(1H)-pyrimidon-dihydrochlorid

Die Synthese von (5R,S)-3,4,5,6-Tetrahydro-5-ethylamino-2-ureidopyrimidin-4-on und die Umsetzung dieses Bausteins mit (3S)-1-Diazo-3-benzyloxycarbonyl-6-[N,N'-bis-(benzyloxycarbonyl)guanidino]hexan-2-on erfolgen analog zu einer publizierten Vorschrift (vgl. V.V. Sokolov, S.I. Kozhushkov, S. Nikolskaya, V.N. Belov, M. Es-Sayed, A. de Meijere, *Eur*. *J*. *Org*. *Chem*. **1998**, 777). Als Ausgangsmeaterial wird N-Ethyl-DL-asparagin verwendet (Y. Liwschitz, Y. Edlitz-Pfeffermann, Y. Lapidoth, *J*. *Am*. *Chem*. *Soc*. **1956**, 78, 3069). Die anschließende Abspaltung der Benzyloxycarbonyl-Schutzgruppen erfolgt wie für Beispiel 1 beschrieben. Die Titelverbindung wird als weißer Feststoff erhalten. Schmelzpunkt: 170-172°C. %). ¹H-NMR (250 MHz, D₂O): 1.03 (t, 3H), 1.35-1.55 (m, 4H), 2.25-2.45 (m, 2H), 2.95-3.05 (m, 2H), 3.30-3.77 (m, 5H), 4.42 (m, 1H). MS (FAB): 370 (M+H)⁺.

### Beispiel 16

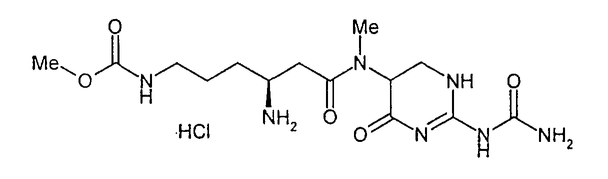

### (3'S,5R,S)-5-[N-Methyl-N-(3'-amino-6'-methoxycarbonylaminohexanoyl)-amino]-5,6-dihydro-2-ureido-4(1H)-pyrimidon-hydrochlorid

Die Kupplung von 104 mg (0.56 mmol) (5R,S)-3,4,5,6-Tetrahydro-5-methylamino-2-ureidopyrimidin-4-on und 190 mg (0.56 mmol) (3S)-3-Benzyloxycarbonylamino-6-methoxycarbonylaminohexansäure sowie die anschließende Abspaltung der Benzyloxycarbonyl-Schutzgruppe erfolgt wie bei Beispiel 1 beschrieben. Die Titelverbindung wird als weißer Feststoff erhalten (30 mg, 13%). ¹ H-NMR (400 MHz, CD₃OD): 1.58 (m, 2H), 1.69 (m, 2H), 2.65-3.07 (m, 4H), 3.14 (m, 3H), 3.57 (m, 1H), 3.63 (s, 3H), 3.88 (m, 1H), 4.03 (m, 1H), 5.18 (m, 1H). MS (ESI): 372 (M+H)⁺.

### Beispiel 17

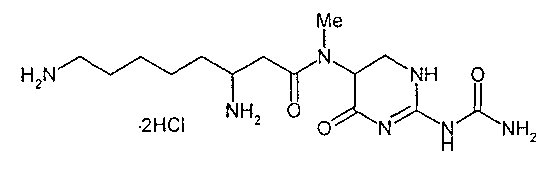

### (3'R,S,5R,S)-5-[N-Methyl-N-(3',8'-diaminooctanoyl)amino]-5,6-dihydro-2-ureido-4(1H)-pyrimidon-dihydrochlorid

3.70 g (14.7 mmol) 6-Benzyloxycarbonylamino-1-hexanol (S. Fernandez, E. Menendez, V. Gotar, *Synthesis* **1991**, 713-716) und 14.9 g (147 mmol) Triethylamin werden in 50 ml Dichlormethan gelöst. Die Lösung wird auf 0°C gekühlt und mit 7.03 g (44.2 mmol) Schwefeltrioxid-Pyridinkomplex in 44 ml Dimethylsulfoxid versetzt. Anschließend wird auf Raumtemperatur erwärmt und 25 min. gerührt. Man gibt die Lösung in 400 ml Eiswasser und extrahiert mehrfach mit Diethylether. Die vereinigten organischen Phasen werden dreimal mit 1 M Salzsäure und je einmal mit Wasser und gesättigter wäßriger NaCl-Lösung gewaschen, über Na₂SO₄ getrocknet und im Vakuum vom Lösungsmittel befreit. Das resultierende farblose Öl (3.50 g) wird in 20 ml THF gelöst (Lösung A). Separat werden 3.54 g (16.9 mmol) Diethylphosphonoessigsäuremethylester in 40 ml THF bei 0°C mit 17 ml einer 1 M THF-Lösung von Natrium(bistrimethylsilyl)amid versetzt. Man rührt 45 min. und gibt Lösung A bei 0°C zu. Die resultierende Lösung wird auf Raumtemperatur erwärmt, 2 h gerührt und im Vakuum eingedampft. Der Rückstand wird an Kieselgel chromatographiert (Essigsäureethylester/Cyclohexan 1:4 bis 1:2). Man erhält 1.73 g (34%) (Z)-8-Benzyloxycarbonylamino-2-octencarbonsäuremethylester als farbloses Öl. ¹H-NMR (200 MHz, DMSO): 1.17-1.49 (m, 6H), 2.18 (q, 2H), 2.95 (q, 2H), 3.66 (s, 3H), 5.00 (s, 2H), 5.87 (d, 1H), 6.89 (td, 1H), 7.25 (m, 1H), 7.34 (m, 5H). MS (DCI/NH₃): 323 (M+NH₄)⁺.

0.88 g (2.9 mmol) des Esters werden zu 9 ml gesättigter ammoniakalischer Ethanollösung gegeben. Im geschlossenen Gefäß wird 6 h auf 100°C (Badtemperatur) erwärmt. Nach Abkühlen auf Raumtemperatur werden die flüchtigen Komponenten im Vakuum entfernt. Der Rückstand wird in 15 ml Dichlormethan aufgenommen. Die resultierende Lösung wird auf 0°C gekühlt und nacheinander mit 0.58 ml (4.2 mmol) Triethylamin und 0.51 ml (3.6 mmol) Chlorameisensäurebenzylester versetzt. Man läßt auf Raumtemperatur erwärmen und rührt 15 h nach. Man verdünnt mit 50 ml Dichlormethan, wäscht mit 1 M Salzsäure, trocknet die organische Phase über Na₂SO₄ und entfernt die flüchtigen Komponenten im Vakuum. Chromatographie des Rückstands an Kieselgel (Essigsäureethylester/Cyclohexan 1:3 bis 1:2) gibt 194 mg (14%) (3R,S)-3,8-Bis(benzyloxycarbonylamino)octansäureethylester [MS (ESI): 471 (M+H)⁺] und 248 mg (19 %) (3R,S)-3,8-Bis(benzyloxycarbonylamino)octansäuremethylester [MS (ESI): 457 (M+H)⁺] in Form farbloser Öle. Die beiden Produkte werden zusammengegeben, in 10 ml Dichlormethan gelöst und mit 280 mg (1.9 mmol) Kaliumtrimethylsilanolat versetzt. Man rührt 1 h bei RT, gibt weitere 100 mg Kaliumtrimethylsilanolat zu und läßt eine weitere Stunde rühren. Man verdünnt mit 20 ml Dichlormethan, wäscht die organische Phase mir 2 M Salzsäure, trocknet über Na₂SO₄ und dampft das Lösungsmittel im Vakuum ab. Man erhält 393 mg (93%) (3R,S)-3,8-Bis(benzyloxycarbonylamino)octansäure als weißen Feststoff. ¹H-NMR (300 MHz, DMSO): 1.21 (m, 4H), 1.38 (m, 4H), 2.36 (m, 2H), 2.95 (q, 2H), 3.77 (m, 1H), 5.01 (s, 4H), 7.14 (m, 1H), 7.35 (m, 10H), 12.08 (s, 1H).

Die Kupplung von 200 mg (0.45 mmol) der so hergestellten Säure mit 84 mg (0.45 mmol) (5R,S)-3,4,5,6-Tetrahydro-5-methylamino-2-ureidopyrimidin-4-on sowie die anschließende Abspaltung der Benzyloxycarbonyl-Schutzgruppen erfolgt wie bei Beispiel 1 beschrieben. Die Titelverbindung wird als weißer Feststoff erhalten (175 mg, 94 %). ¹H-NMR (400 MHz, CD₃OD): 1.47 (m, 4H), 1.72 (m, 4H), 2.75 (m, 1H), 2.94 (m, 3H), 3.15 (m, 3H), 3.58 (m, 1H), 3.90 (m, 1H), 4.03 (m, 1H), 5.18 (m, 1H). MS (ESI): 342 (M+H)⁺.

### Beispiel 18

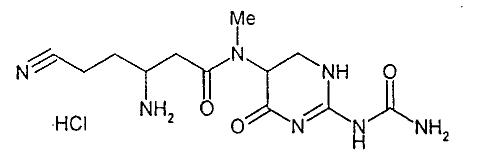

### (3'R,S,5R,S)-5-[N-Methyl-N-(3'-amino-5'-cyanopentanoyl)amino]-5,6-dihydro-2-ureido-4(1H)-pyrimidon-hydrochlorid

Eine Lösung von 1.00 g (10.1 mmol) 3-Cyanopropansäure-Natriumsalz in 50 ml Dichlormethan wurde mit 30 ml einer 1 molaren Salzsäure extrahiert. Die organsiche Phase wurde über Magnesiumsulfat getrocknet und das Lösungsmittel wurde am Rotationsverdampfer abdestilliert. Die rohe 3-Cyanopropansäure wurde im Hochvakuum von Lösungsmittelresten befreit.

Es wurde in 15 ml THF aufgenommen und bei 0°C mit 1.96 g (12.1 mmol) N,N-Carbonyldiimidazol portionsweise versetzt. Man ließ eine Stunde bei Raumtemperatur nachrühren (Lösung A).

In einem zweiten Kolben wurde eine Lösung von 960 mg (10.1 mmol) Magnesiumchlorid und 2.75 g (15.1 mmol) Malonsäureethylester-Kaliumsalz in 25 ml THF für 4 Stunden auf 50°C erwärmt. Es wurde auf Raumtemperatur abgekühlt und anschließend tropfenweise mit der zuvor hergestellten Lösung A versetzt. Man ließ über Nacht bei Raumtemperatur rühren.

Das Lösungsmittel wurde am Rotationsverdampfer abdestilliert und der Rückstand wurde in 20 ml Wasser und 50 ml Dichlormethan aufgenommen. Die organische Phase wurde über Kieselgur filtriert und über Natriumsulfat getrocknet. Der Rückstand wurde über eine Flash-Säule gereinigt (Kieselgel, Laufmittel Cyclohexan/Essigester 10:1 polarer werdend auf 1:1). Es wurden 617 mg (36%) 5-Cyano-3-oxopentansäureethylester erhalten. ¹H-NMR (300 MHz, DMSO) 1.19 (t, 3H), 2.60 (t, 2H), 2.95 (t, 2H), 3.62 (s, 2H), 4.10 (q, 2H). MS (EI): 169 (M)⁺.

Es wurden 3.80 g (22.4 mmol) 5-Cyano-3-oxopentansäureethylester in 5 ml einer gesättigten ethanolische Ammoniak-Lösung aufgenommen und 24 h bei Raumtemperatur gerührt. Die flüchtigen Bestandteile wurden am Rotationsverdampfer abdestilliert und man erhielt 3.60 g (95 %) 3-Amino-5-cyano-2-pentensäureethylester. ¹H-NMR (300 MHz, DMSO) 1.16 (t, 3H), 2.37 (t, 2H), 2.75 (t, 2H), 3.99 (q, 2H), 4.41 (s, 1H), 6.96 (s, breit, 1H), 7.69 (s, breit, 1H). MS (DCI/NH₃): 169 (M+H)⁺, 186 (M+ NH₄)⁺, 337 (2M+H)⁺.

Bei 0°C wurde zu einer Lösung von 56.0 mg (892 µmol) Natriumcyanoborhydrid in 1 ml abs. Methanol eine Lösung von 50.0 mg (297 µmol) 3-Amino-5-cyano-2-pentensäure-ethylester in 1 ml Methanol tropfenweise zugegeben. Man versetzte mit 6 Tropfen Eisessig und entfernte das Kühlbad und ließ 2 h bei Raumtemperatur nachrühren.

Es wurde mit 1 ml einer ges. Natriumhydrogencarbonat-Lösung versetzt und am Rotationsverdampfer eingeengt. Die wässrige Phase wurde zweimal mit je 5 ml Dichlormethan extrahiert. Die organische Phase wurde über Natriumsulfat getrocknet und das Lösungsmittel wurde am Rotationsverdampfer abdestilliert. Es verblieben 39.9 mg (79%) des gewünschten 3-Amino-5-cyanopentansäureethylester. ¹H-NMR (300 MHz, DMSO) 1.19 (t, 3H), 1.49 (m, 1H), 1.68 (m, 1H), 2.27 (dd, 1H), 2.40 (dd, 1H), 2.55 (t, 2H), 3.00 (m, 1 H), 4.08 (q, 2H). MS (DCI/NH₃): 171 (M+H)⁺.

Zu einer Lösung von 470 mg (2.76 mmol) 3-Amino-5-cyanopentansäureethylester und 458 mg (3.31 mmol) Kaliumcarbonat in 10 ml Dioxan/Wasser (1:1) wurde bei Raumtemperatur eine Lösung von 723 mg (3.31 mmol) BOC-Anhydrid in 0.5 ml Dioxan zugetropft. Die flüchtigen Bestandteile wurden am Rotationsverdampfer abdestilliert und der Rückstand wurde zweimal mit je 5 ml Dichlormethan extrahiert. Die organische Phase wurde über Natriumsulfat getrocknet und das Lösungsmittel wurde am Rotationsverdampfer abdestilliert. Das Rohprodukt wurde über eine Flash-Säule gereinigt (Kieselgel, Laufmittel: Cyclohexan/Essigester 20:1 polarer werdend auf 1:1). Man erhielt 505 mg (68%) 3-[(*tert*-butoxycarbonyl)amino]-5-cyanopentansäureethylester. ¹H-NMR (300 MHz, DMSO) 1.19 (t, 3H), 1.35 (s, 9H), 1.70 (m, 2H), 2.48 (m, 4H), 3.81 (m, 1H), 4.02 (q, 2H), 6.80 (m, 1H). MS (DCI/NH₃): 288 (M+NH₄)⁺.

Zu einer Lösung von 300 mg (1.11 mmol) 3-[(*tert*-butoxycarbonyl)amino]-5-cyanopentansäureethylester in 1 ml Dichlormethan wurden 213 mg (1.66 mmol) Kaliumtrimethylsilanolat gegeben und es wurde bei Raumtemperatur gerührt. Nach 2 Stunden wurden erneut 213 mg ) Kaliumtrimethylsilanolat zugesetzt und es wurde 30 min nachgerührt.

Man versetzte mit 1 ml einer ges. Ammoniumchlorid-Lösung und extrahierte mit 2 ml Dichlormethan. Die wässrige Pahse wurde mit 1 molarer Slzsäure auf pH 1 gestellt und zweimal mit je 3 ml Dichlormethan extrahiert. Die vereinigten organischen Phasen wurden über Natriumsulfat getrocknet und am Rotationsverdampfer vom Lösungsmittel befreit. Man erhielt 177 mg (66 %) der gewünschten 3-[(*tert*butoxycarbonyl)amino]-5-cyanopentansäure. ¹H-NMR (300 MHz, d⁶-DMSO) 1.38 (s, 9H), 1.65 (m , 1H), 1.75 (m, 1H), 2.38 (m, 2H), 2.45 (m, 2H), 3.79 (m, 1H), 6.80 (d, 1H), 12.20 (s, breit, 1H). MS (DCI/NH₃): 260 (M+NH₄)⁺.

Die Kupplung von 15 mg (82 µmol) 3-[(*tert*-butoxycarbonyl)amino]-5-cyanopentansäure mit (5R,S)-3,4,5,6-Tetrahydro-5-methylamino-2-ureidopyrimidin-4-on erfolgte wie bei Beispiel 1 beschrieben. Die Ausbeute betrug 32%. Zur Abspaltung der BOC-Gruppe nahm man das Rohprodukt in 1 ml 4 molarer HCl in Dioxan auf und rührte 30 min bei Raumtemperatur. Alle flüchtigen Bestandteile wurden am Rotationsverdampfer abdestilliert. Der Rückstand wurde in Methanol aufgenommen und tropfenweise mit Aceton versetzt bis ein Niederschlag auftrat. Es wurde dekantiert und der verbleibende weiße Feststoff wurde im Ölpumpenvakuum von Lösungsmittelresten befreit. Man erhielt 2.1 mg (28%) der Titelverbindung. MS (DCI): 311 (M+H)⁺.

## Patentansprüche

1. Verbindungen der allgemeinen Formel: 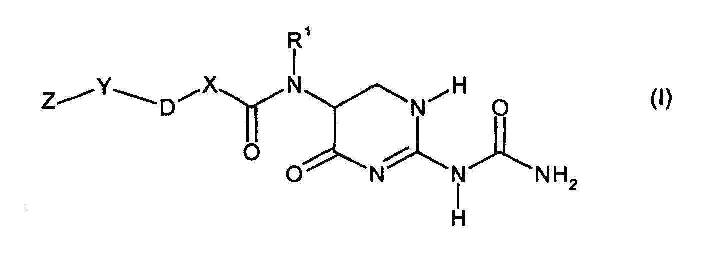 worin
R¹ Wasserstoff oder (C₁-C₆)Alkyl ist,
X eine Gruppe der Formel -(CH₂)ₘ- darstellt, worin m 0, 1 oder 2 ist,
D ausgewählt wird aus Gruppen der Formeln D₁ bis D₃ 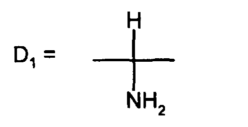 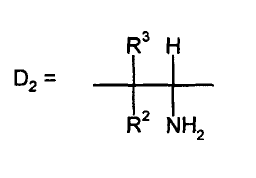 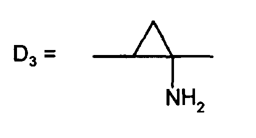 worin
R² Wasserstoff oder Hydroxy ist,
R³ Wasserstoff ist, oder
R² und R³ zusammen eine Oxogruppe bilden,
Y eine geradkettige oder verzweigtkettige (C₁-C₅)Alkandiyl-Gruppe, die gegebenenfalls durch Hydroxy oder Oxo substituiert sein kann, oder eine Gruppe der folgenden Formel 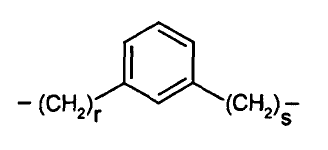 darstellt, worin r und s gleich oder verschieden sind und 0, 1 oder 2 sind,
Z eine Gruppe darstellt, die ausgewählt wird aus Gruppen der Formel 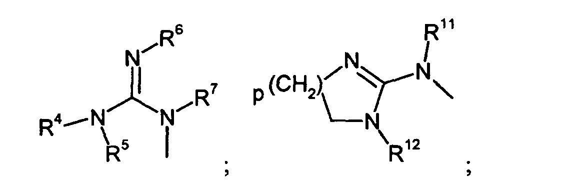 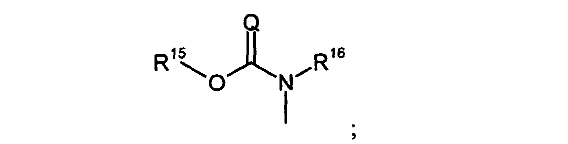 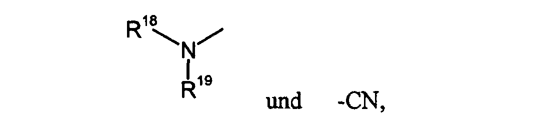 worin R⁴, R⁵, R⁶, R⁷, R¹¹, R¹², R¹⁵, R¹⁶, R¹⁸ und R¹⁹ jeweils unabhängig voneinander ausgewählt werden aus der Gruppe, die aus Wasserstoff, (C₁-C₆)Alkyl, (C₁-C₄)Alkanoyl, t-Butoxycarbonyl, Benzyloxycarbonyl und Benzyl besteht,
Q Sauerstoff oder Schwefel darstellt und
p 1, 2, oder 3 darstellt und
mit Ausnahme der Verbindungen, worin
R¹ Methyl ist, m 1 ist, D D₁ ist, Y -(CH₂)₃- ist, und Z eine Gruppe der Formel 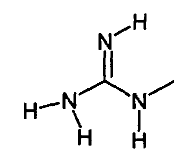 ist,
und pharmazeutisch verträgliche Salze davon.

2. Verbindungen nach Anspruch 1 der allgemeinen Formel (I),
worin
R¹ Wasserstoff oder (C ₁-C₆)Alkyl ist,
X eine Gruppe der Formel -(CH₂)ₘ- darstellt, worin m 0, 1 oder 2 ist,
D ausgewählt wird aus Gruppen der Formeln D₁ bis D₃ 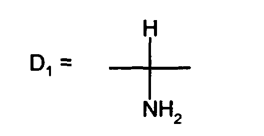 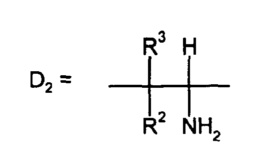 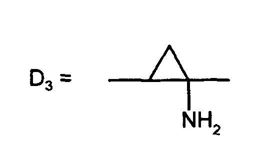 worin
R² Wasserstoff oder Hydroxy ist,
R³ Wasserstoff ist, oder
R² und R³ zusammen eine Oxogruppe bilden,
Y eine geradkettige oder verzweigtkettige (C₁-C₅)Alkandiyl-Gruppe, die gegebenenfalls durch Hydroxy oder Oxo substituiert sein kann, oder eine Gruppe der folgenden Formel 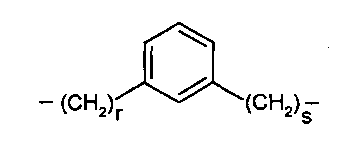 darstellt, worin r und s gleich oder verschieden sind und 0, 1 oder 2 sind,
Z eine Gruppe darstellt, die ausgewählt wird aus Gruppen der Formel 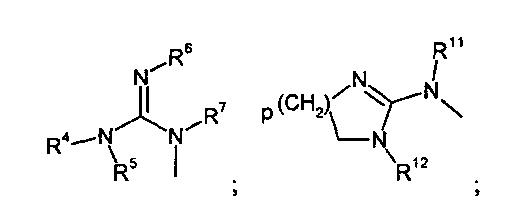 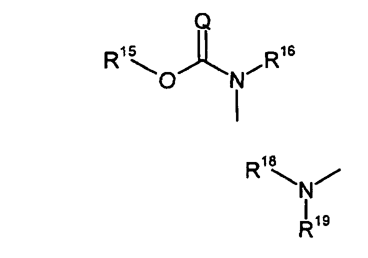 und
worin R⁴, R⁵, R⁶, R⁷, R¹¹, R¹², R¹⁵, R¹⁶, R¹⁸ und R¹⁹ jeweils unabhängig voneinander ausgewählt werden aus der Gruppe, die aus Wasserstoff, (C₁-C₆)Alkyl, (C₁-C₄)Alkanoyl, t-Butoxycarbonyl, Benzyloxycarbonyl und Benzyl besteht,
Q Sauerstoff oder Schwefel darstellt und
p 1, 2, oder 3 darstellt und
mit Ausnahme der Verbindungen, worin
R¹ Methyl ist, m 1 ist, D D₁ ist, Y -(CH₂)₃- ist, und Z eine Gruppe der Formel 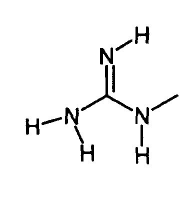 ist,
und pharmazeutisch verträgliche Salze davon.

3. Verbindungen nacxh Anspruch 1 oder 2, worin m 1 oder 2 ist.

4. Verbindungen nach Anspruch 1, 2 oder 3, worin Y eine geradkettige oder verzweigtkettige (C₁-C₅)Alkandiyl-Gruppe ist.

5. Verbindungen nach irgend einem der Ansprüche 1 bis 4, worin Y m-Phenylen ist.

6. Verbindungen nach irgend einem der Ansprüche 1 bis 5, worin Z eine Gruppe der Formel 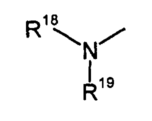 darstellt, worin R¹⁸ und R¹⁹ wie im Anspruch 1 definiert sind.

7. Verbindungen nach irgend einem der Ansprüche 1 bis 5, worin Z eine Gruppe der Formel 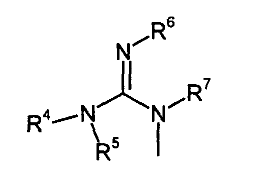 darstellt, worin R⁴, R⁵, R⁶ und R⁷ wie im Anspruch 1 definiert sind.

8. Verbindungen nach irgend einem der Ansprüche 1 bis 7, worin D eine Gruppe der Formel 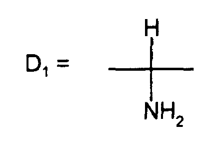 ist.

9. Verbindungen nach irgend einem der Ansprüche 1 bis 7, worin D eine Gruppe der Formel 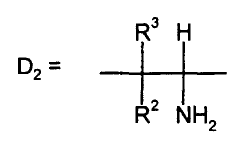 ist.

10. Verbindungen nach irgend einem der Ansprüche 1 bis 7, worin D eine Gruppe der Formel 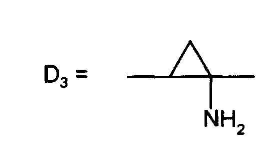 ist.

11. Verbindung nach Anspruch 1 oder 2 der Formel 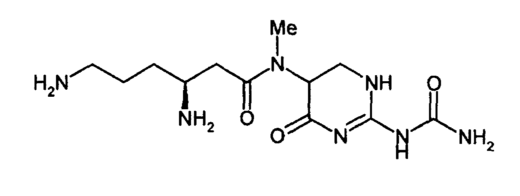 und deren pharmazeutisch verträgliche Salze.

12. Verbindung nach Anspruch 1 oder 2 der Formel 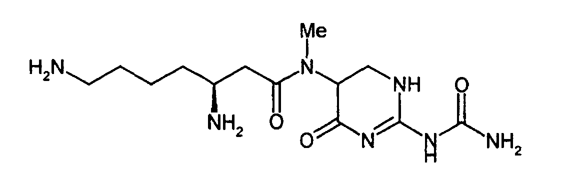 und deren pharmazeutisch verträgliche Salze.

13. Verfahren zur Herstellung der Verbindungen nach Anspruch 1, **dadurch gekennzeichnet, daß** man Verbindungen der Formel (II) 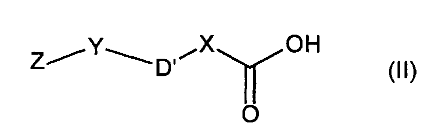 worin X, Y und Z wie im Anspruch 1 definiert sind und D' ausgewählt wird aus Gruppen der Formeln D'₁ bis D'₃ 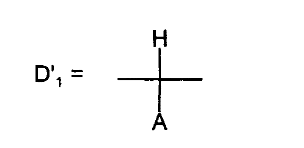 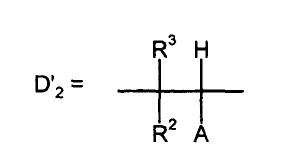 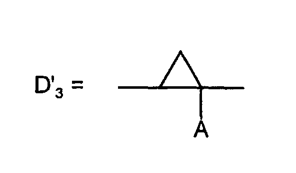 worin R² und R³ wie im Anspruch 1 sind und A eine konventionell geschützte Aminogruppe ist, mit Verbindungen der Formel (III) 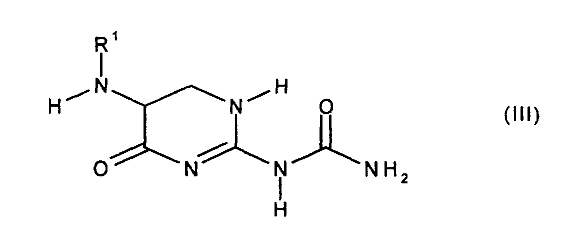 worin R¹ wie im Anspruch 1 definiert ist, in Gegenwart von Kupplungsmitteln, wahlweise in Gegenwart von Basen, umsetzt und in der geschützten Aminogruppe A die konventionelle Schutzgruppe nach an sich bekannten Verfahren abspaltet.

14. Verfahren nach Anspruch 13, worin das Kupplungsmittel ausgewählt wird aus [O-(7-Azabenzotriazol-1-yl)-1,1,3,3-tetramethyluronium]hexafluorophosphat (HATU) oder [Bromo-tris-pyrrolidino-phosphonium]hexafluorophosphat (PyBroP).

15. Verbindungen nach Anspruch 1 oder 2 zur Verwendung als Arzneimittel.

16. Pharmazeutische Zusammensetzung, die eine Verbindung nach Anspruch 1 oder 2 zusammen mit pharmazeutisch verträglichen Trägern oder Exzipienten umfaßt.

17. Verwendung einer Verbindung nach Anspruch 1 oder 2 für die Herstellung eines Arzneimittels.

18. Verwendung einer Verbindung nach Anspruch 1 oder 2 für die Herstellung eines Arzneimittels zur Behandlung und Prävention bakterieller Infektionen bei Menschen oder Tieren.

## Claims

1. Compounds of the general formula: 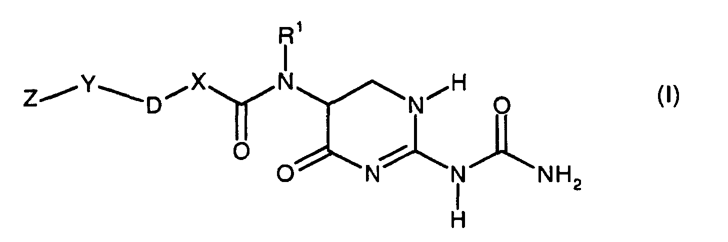 in which
R¹ represents hydrogen or (C₁-C₆)alkyl,
X represents a group of the formula -(CH₂)ₘ-, in which m is 0, 1 or 2,
D is selected from groups of the formulae D₁ to D₃ 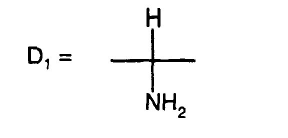 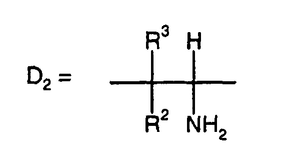 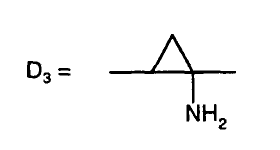 in which
R² represents hydrogen or hydroxyl,
R³ represents hydrogen, or
R² and R³ together form an oxo group,
Y represents a straight-chain or branched (C₁-C₅)alkanediyl group which may optionally be substituted by hydroxyl or oxo, or represents a group of the formula below 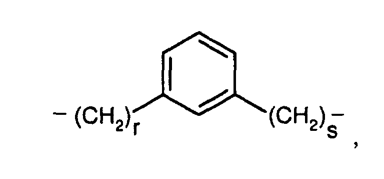 in which r and s are identical or different and are 0, 1 or 2,
Z represents a group selected from groups of the formulae 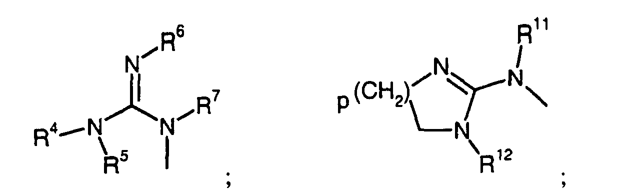 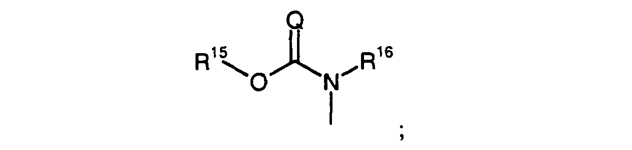 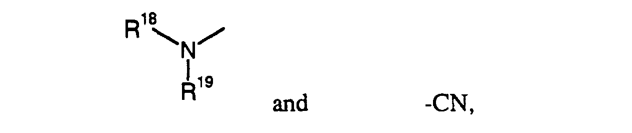 in which R⁴, R⁵, R⁶, R⁷, R¹¹, R¹², R¹⁵, R¹⁶, R¹⁸ and R¹⁹ in each case independently of one another are selected from the group consisting of hydrogen, (C₁-C₆)alkyl, (C₁-C₄)alkanoyl, t-butoxycarbonyl, benzyloxycarbonyl and benzyl,
Q represents oxygen or sulphur and
p is 1, 2 or 3
except for compounds in which
R¹ represents methyl, m is 1, D represents D₁, Y represents -(CH₂)₃-and Z represents a group of the formula 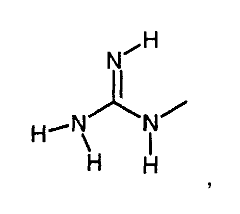
and pharmaceutically acceptable salts thereof.

2. Compounds according to Claim 1 of the general formula (I),
in which
R¹ represents hydrogen or (C₁-C₆)alkyl,
X represents a group of the formula -(CH₂)ₘ-, in which m is 0, 1 or 2,
D is selected from groups of the formulae D₁ to D₃ 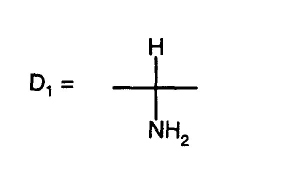 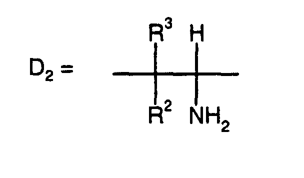 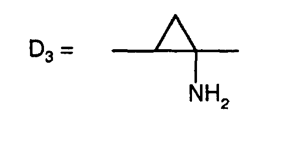 in which
R² represnets hydrogen or hydroxyl,
R³ represents hydrogen, or
R² and R³ together form an oxo group,
Y represents a straight-chain or branched (C₁-C₅)alkanediyl group which may optionally be substituted by hydroxyl or oxo, or represents a group of the formula below 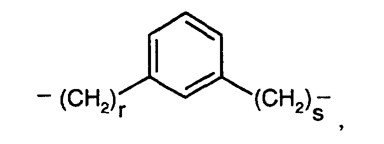 in which r and s are identical or different and are 0, 1 or 2,
Z represents a group selected from groups of the formulae 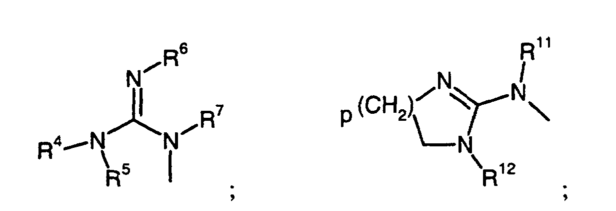 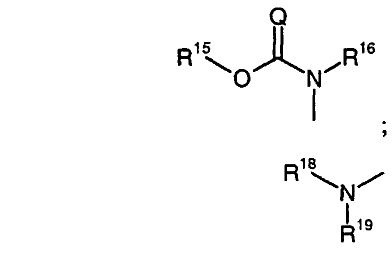 and
in which R⁴, R⁵, R⁶, R⁷, R¹¹, R¹², R¹⁵, R¹⁶, R¹⁸ and R¹⁹ in each case independently of one another are selected from the group consisting of hydrogen, (C₁-C₆)alkyl, (C₁-C₄)alkanoyl, t-butoxycarbonyl, benzyloxycarbonyl and benzyl,
Q represents oxygen or sulphur and
p is 1, 2 or 3,
except for compounds in which
R¹ represents methyl, m is 1, D represents D₁, Y represents -(CH₂)₃- and Z represents a group of the formula 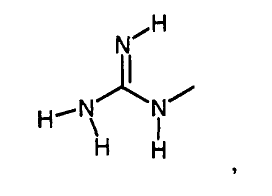
and pharmaceutically acceptable salts thereof.

3. Compounds according to Claim 1 or 2, in which m is 1 or 2.

4. Compounds according to Claim 1, 2 or 3, in which Y represents a straight-chain or branched (C₁-C₅)alkanediyl group.

5. Compounds according to any of Claims 1 to 4, in which Y represents m-phenylene.

6. Compounds according to any of Claims 1 to 5, in which Z represents a group of the formula 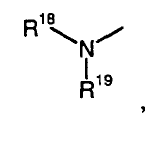 in which R¹⁸ and R¹⁹ are as defined in Claim 1.

7. Compounds according to any of Claims 1 to 5, in which Z represents a group of the formula 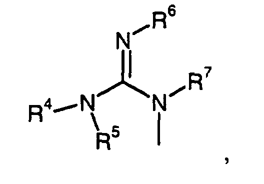 in which R⁴, R⁵, R⁶ and R⁷ are as defined in Claim 1.

8. Compounds according to any of Claims 1 to 7, in which D represents a group of the formula

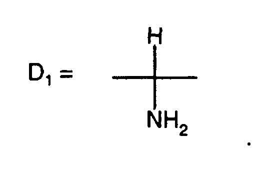

9. Compounds according to any of Claims 1 to 7, in which D represents a group of the formula

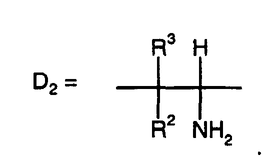

10. Compounds according to any of Claims 1 to 7, in which D represents a group of the formula

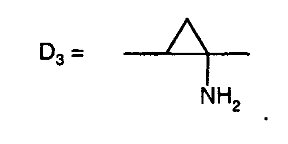

11. Compound according to Claim 1 or 2 of the formula 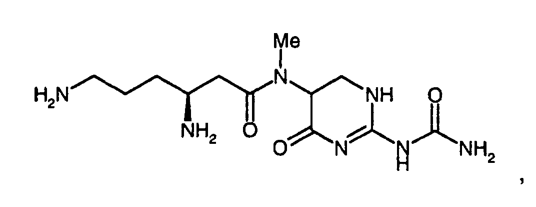 and pharmaceutically acceptable salts thereof.

12. Compound according to Claim 1 or 2 of the formula 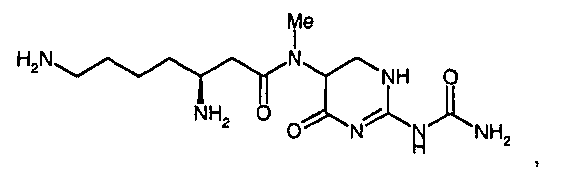 and pharmaceutically acceptable salts thereof.

13. Process for preparing compounds according to Claim 1, **characterized in that** compounds of the formula (II) 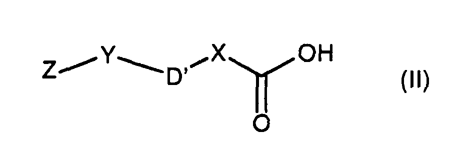 in which X, Y and Z are as defined in Claim 1 and D' is selected from groups of the formulae D'₁ to D'₃ 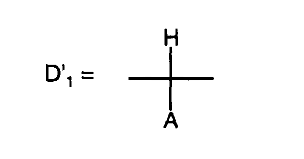 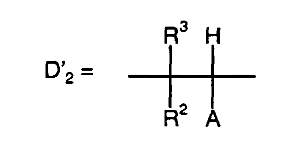 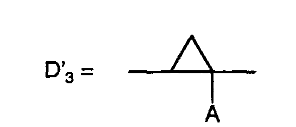 in which R² and R³ are as defined in Claim 1 and A is a conventionally protected amino group are reacted with compounds of the formula (III) 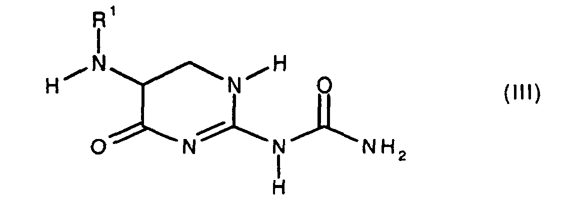 in which R¹ is as defined in Claim 1, in the presence of coupling agents, optionally in the presence of bases, and the conventional protective group in the protected amino group A is removed by processes known per se.

14. Process according to Claim 13, in which the coupling agent is selected from O-(7-azabenzotriazol-1-yl)-1,1,3,3-tetramethyluronium hexafluorophosphate (HATU) or bromo-tris-pyrrolidino-phosphonium hexafluorophosphate (PyBroP).

15. Compounds according to Claim 1 or 2 for use as medicaments.

16. Pharmaceutical composition, comprising a compound according to Claim 1 or 2 together with pharmacetically acceptable carriers or excipients.

17. Use of a compound according to Claim 1 or 2 for preparing a medicament.

18. Use of a compound according to Claim 1 or 2 for preparing a medicament for treating and preventing bacterial infection in humans or animals.

## Revendications

1. Composés de formule générale : 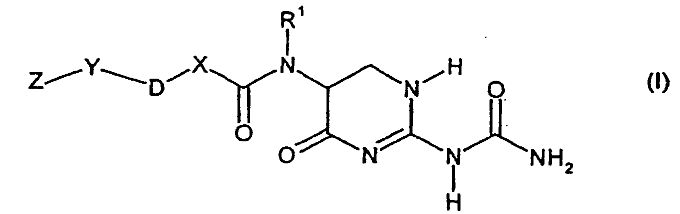 dans laquelle
R¹ représente l'hydrogène ou un reste alkyle en C₁ à C₆,
X est un groupe de formule -(CH₂)ₘ- dans laquelle m a la valeur 0, 1 ou 2,
D est choisi parmi des groupes de formules D₁ à D₃ ; 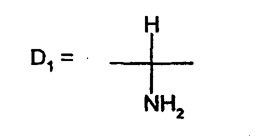 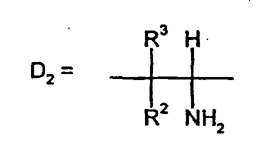 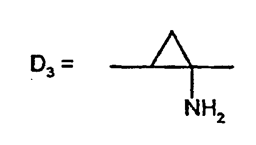 où
R² désigne l'hydrogène ou le radical hydroxy,
R³ est l'hydrogène, ou bien
R² et R³ forment ensemble un groupe oxo,
Y représente un groupe alcanediyle en C₁ à C₅, à chaîne droite ou à chaîne ramifiée, qui peut éventuellement porter un substituant hydroxy ou oxo, ou représente un groupe de formule suivante 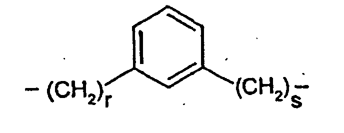 dans laquelle r et s sont égaux ou différents et ont la valeur 0, 1 ou 2,
Z représente un groupe qui est choisi parmi des groupes de formules 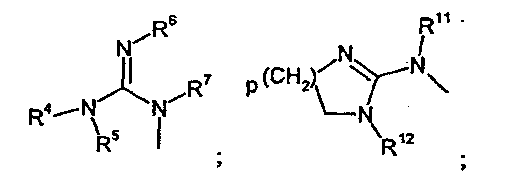 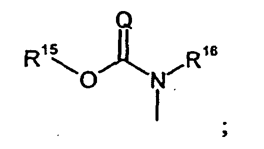 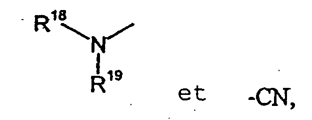 où R⁴, R⁵, R⁶, R⁷, R¹¹, R¹², R¹⁵, R¹⁶, R¹⁸ et R¹⁹ sont choisis chacun, indépendamment les uns des autres, dans le groupe constitué de l'hydrogène et des restes alkyle en C₁ à C₆, alcanoyle en C₁ à C₄, tertiobutoxycarbonyle, benzyloxycarbonyle et benzyle,
Q représente l'oxygène ou le soufre, et
p a la valeur 1, 2 ou 3,
à l'exception des composés dans lesquels
R¹ est un reste méthyle, m est égal à 1, D est le groupe D₁, Y représente -(CH₂)₃-, et Z est un groupe de formule 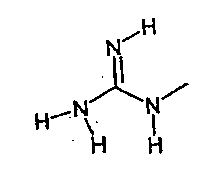
et leurs sels acceptables du point de vue pharmaceutique.

2. Composés suivant la revendication 1, de formule générale (I),
dans laquelle
R¹ représente l'hydrogène ou un reste alkyle en C₁ à C₆,
X est un groupe de formule -(CH₂)ₘ-, dans laquelle m a la valeur 0, 1 ou 2,
D est choisi parmi des groupes de formules D₁ à D₃ 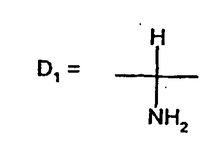 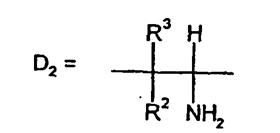 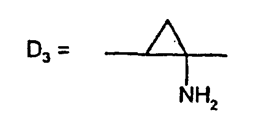 où
R² est l'hydrogène ou le radical hydroxy,
R³ est l'hydrogène, ou bien
R² et R³ forment ensemble un groupe oxo,
Y est un groupe alcanediyle en C₁ à C₅ à chaîne droite ou à chaîne ramifiée, qui peut éventuellement porter un substituant hydroxy ou oxo, ou représente un groupe de formule suivante 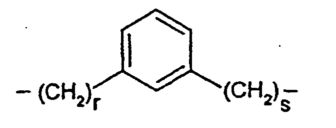 dans laquelle r et s sont égaux ou différents et ont la valeur 0, 1 ou 2,
Z représente un groupe qui est choisi parmi des groupes de formules 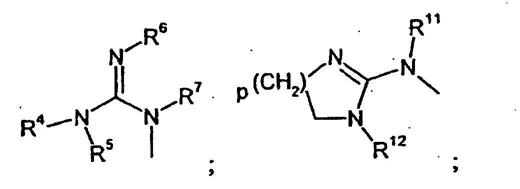 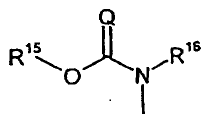 et 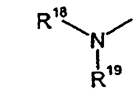 où R⁴, R⁵, R⁶, R⁷, R¹¹, R¹², R¹⁵, R¹⁶, R¹⁸ et R¹⁹ sont choisis chacun, indépendamment les uns des autres, dans le groupe qui est constitué de l'hydrogène et des restes alkyle en C₁ à C₆, alcanoyle en C₁ à C₄, tertio-butoxycarbonyle, benzyloxycarbonyle et benzyle,
Q représente l'oxygène ou le soufre et
p a la valeur 1, 2 ou 3,
à l'exception des composés dans lesquels
R¹ est le reste méthyle, m est égal à 1, D représente D₁, Y est un groupe -(CH₂)₃-, et Z est un groupe de formule 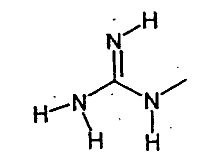
et leurs sels acceptables du point de vue pharmaceutique.

3. Composés suivant la revendication 1 ou 2, dans lesquels m a la valeur 1 ou 2.

4. Composés suivant la revendication 1, 2 ou 3, dans lesquels Y représente un groupe alcanediyle en C₁ à C₅ à chaîne droite ou à chaîne ramifiée.

5. Composés suivant l'une quelconque des revendications 1 à 4, dans lesquels Y est le groupe m-phénylène.

6. Composés suivant l'une quelconque des revendications 1 à 5, dans lesquels Z est un groupe de formule 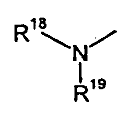 où R¹⁸ et R¹⁹ sont tels que définis dans la revendication 1.

7. Composés suivant l'une quelconque des revendications 1 à 5, dans lesquels Z est un groupe de formule 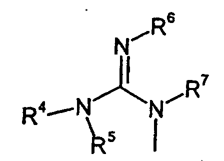 où R⁴, R⁵, R⁶ et R⁷ sont tels que définis dans la revendication 1.

8. Composés suivant l'une quelconque des revendications 1 à 7, dans lesquels D est un groupe de formule

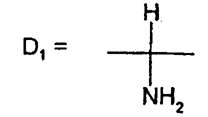

9. Composés suivant l'une quelconque des revendications 1 à 7, dans lesquels D est un groupe de formule

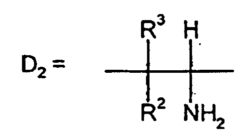

10. Composés suivant l'une quelconque des revendications 1 à 7, dans lesquels D est un groupe de formule

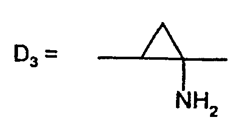

11. Composé suivant la revendication 1 ou 2, de formule 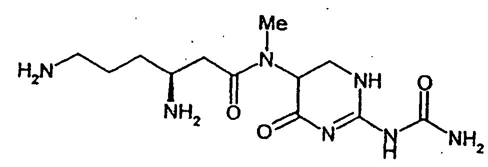 et ses sels acceptables du point de vue pharmaceutique.

12. Composé suivant la revendication 1 ou 2, de formule 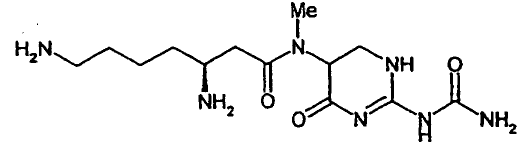 et ses sels acceptables du point de vue pharmaceutique.

13. Procédé de production des composés suivant la revendication 1, **caractérisé en ce qu'**on fait réagir des composés de formule (II) 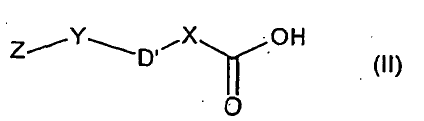 dans laquelle X, Y et Z sont tels que définis dans la revendication 1 et D' est choisi parmi des groupes de formules D'₁ à D'₃ 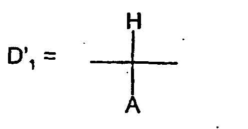 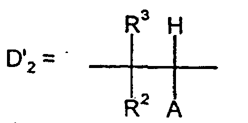 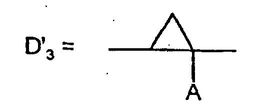 où R² et R³ sont tels que définis dans la revendication 1 et A est un groupe amino protégé de façon classique, avec des composés de formule (III) 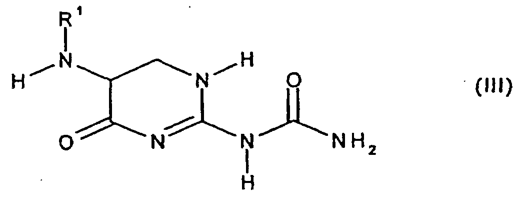 dans laquelle R¹ est tel que défini dans la revendication 1, en présence d'agents de couplage, éventuellement en présence de bases, et on élimine selon des procédés connus le groupe protecteur classique porté par le groupe amino A protégé.

14. Procédé suivant la revendication 13, dans lequel l'agent de couplage est choisi entre l'hexafluorophosphate de [O-(7-azabenzotriazole-1-yl)1,1,3,3-tétraméthyluronium] (HATU) et l'hexafluorophosphate de [bromo-tris-pyrrolidino-phosphonium] (PyBroP).

15. Composés suivant la revendication 1 ou 2, destinés à être utilisés comme médicament.

16. Composition pharmaceutique, qui comprend un composé suivant la revendication 1 ou 2 conjointement avec des supports ou excipients acceptables du point de vue pharmaceutique.

17. Utilisation d'un composé suivant la revendication 1 ou 2 pour la préparation d'un médicament.

18. Utilisation d'un composé suivant la revendication 1 ou 2 pour la préparation d'un médicament destiné au traitement curatif et préventif d'infections bactériennes chez l'homme ou les animaux.
